# EUROPEAN PATENT SPECIFICATION

(11) **EP 1 411 952 B1**
(45) Date of publication and mention of the grant of the patent: **22.04.2009**
(21) Application number: 02751212.8
(22) Date of filing: 28.06.2002
(51) Int. Cl.: A61K 31/702, A61P 1/04, A61P 31/04

(54) **USE OF AT LEAST ONE GLYCOINHIBITOR SUBSTANCE AGAINST INFECTIOUS DISEASES**
VERWENDUNG MINDESTENS EINER GLYCOINHIBITOR-SUBSTANZ GEGEN INFEKTIONSKRANKHEITEN
UTILISATION D'AU MOINS UNE SUBSTANCE GLYCO-INHIBITRICE CONTRE LES MALADIES CONTAGIEUSES

(30) Priority: 29.06.2001 FI 20011402; 29.06.2001 FI 20011403
(43) Date of publication of application: 28.04.2004
(73) Proprietor: Glykos Finland Oy, 00790 Helsinki (FI)
(72) Inventor: NATUNEN, Jari, FIN-01520 Vantaa (FI); MILLER-PODRAZA, Halina, S-421 37 Västra Frölunda (SE); TENEBERG, Susann, S-430 63 Hindas (SE); ANGSTRÖM, Jonas, S-416 57 Göteborg (SE); KARLSSON, Karl-Anders, S-411 43 Göteborg (SE)
(74) Representative: Karvinen, Leena Maria
(86) International application number: PCT/FI2002/000574
(87) International publication number: WO 2003/002127

(56) References cited:
- WO-A1-00/56343
- WO-A1-01/43751
- WO-A1-94/18986
- WO-A1-97/41875
- US-A- 4 859 769
- US-A- 5 217 715
- US-A- 5 242 800
- US-A- 5 386 027
- US-A- 5 514 660
- US-A- 6 136 790
- HOLMES ERIC H. ET AL.: 'Characterization of two monoclonal antibodies specific for lacto-series type 1 chain Gal beta 1 - 3GlcNAc-terminal structures' ARCHIVES OF BIOCHEMISTRY BIOPHYSICS vol. 277, 1990, pages 181 - 188, XP002938388
- MILLER-PODRAZA HALINA ET AL.: 'Binding of helicobacter pylori to sialic acid-containing glycolipids of various origins separated on thin-layer chromatograms' INFECTION AND IMMUNITY vol. 65, no. 6, 1997, pages 2480 - 2482, XP002938383
- FALK PER ET AL.: 'Bacteria of the human intestinal microbiota produce glycosidases specific for lacto-series glycosphingolipids' J. BIOCHEM. vol. 108, 1990, pages 466 - 474, XP002938385
- MAAN-ABUL-MILH ET AL.: 'In vitro binding of helicobacter pylori to monohexosylceramides' GLYCOCONJUGATE JOURNAL vol. 18, 2001, pages 253 - 260, XP002950264
- SIMON P.M. ET AL.: 'Inhibition of helicobacter pylori binding to gastrointestinal epithelial cells by sialic acid-containing oligosaccharides' INFECTION AND IMMUNITY vol. 65, no. 2, 1997, pages 750 - 757, XP002972942
- MASAYUKI MIYAKE ET AL.: 'Human IgG3 monoclonal antibody directed to an unbranched repeating type 2 chain (Gal beta 1 - 4GlcNAc beta1- 3Gal beta 1- 4GlcNAc beta 1 - 3Gal beta 1 - R) which is highly expressed in colonic and hepatocellular carcinoma 1' CANCER RESEARCH vol. 49, 1989, pages 5689 - 5695, XP002920119
- MCNICHOLL IAN R. ET AL.: 'Neuraminidase inhibitors: Zanamivir and oseltamivir' THE ANNALS OF PHARMACOTHERAPY vol. 35, 2001, pages 57 - 70, XP002972943
- CHOUNG U. KIM ET AL.: 'Review: Neuraminidase inhibitors as anti-influenza virus agents' ANTIVIRAL CHEMISTRY & CHEMOTHERAPY vol. 10, 1999, pages 141 - 154, XP000881780

## Description

### FIELD OF THE INVENTION

The present invention describes a pathobiologic process where receptors for pathogenic agents are formed by degradation of glycoconjugates in a patient. The receptors are specific neutral oligosaccharide sequences present on natural glycoconjugates such as glycoproteins and glycolipids. The pathogenic agents can be bacteria, fungi, viruses, or toxins. The invention describes compositions and reagents to treat diseases and conditions due to said pathologic process. The present invention especially relates to compositions and methods to prevent formation of a novel oligosaccharide receptor for *Helicobacter pylori.*

### BACKGROUND OF THE INVENTION

Many, if not most or all, pathogens bind to specific oligosaccharide sequences on animal cell surfaces. The animal cells try to change the cell surface glycosylations, especially the terminal ones to prevent such bindings. The present invention teaches that pathogens use specific glycosidase enzymes to remove protecting modification. The present invention relates to the use of glycosidase inhibitors to prevent the degradation of the animal cell surface glycoconjugates and thus the pathogenic adhesion.

Several neutral and relatively rare oligosaccharide sequences are present on animal and human glycoconjugates. The saccharide sequences of asialo-GM1 (Galβ3GalNAcβ4Galβ4GlcβCer) and asialo-GM2 (GalNAcβ4Galβ4GlcβCer) glycosphingolipids are known receptors for numerous pathogenic agents including many pathogenic bacteria present in lungs, and viruses such as rotavirus.

US patents by Krivan, et al. 5,386,027 (January 1995) and 5,217,715 (June 1993) describe use of asialo-GM1 and asialo-GM2 to inhibit several pathogenic bacteria. The patent applications also suggest the pathogenic mechanism where desialylation by influenza virus creates the unsubstituted receptor GalNAcβ4Gal. However, in such a structure GalNAc is not known to be sialylated and when Gal is α3-sialylated the structure is resistant to most sialidases by steric hindrance. Desialylation of other structures such as the terminal Gal of GMlb (NeuNAcα3Galβ3GalNAcβ4Galβ4GlcβCer) is more likely to happen. These saccharide sequences are also receptors for viruses such as rotavirus (Delorme *et al.* 2001). However, no use of glycosidase inhibitor is described.

Lactosylceramide is also a common receptor for pathogenic bacteria, it has also been shown to bind cell surface polysaccharide present on pathogenic yeasts. Several viruses including human influenza virus, rotavirus, reovirus, mumps virus and rabies virus bind to glycolipids containing an oligosaccharide sequence up to tetrasaccharide as exemplified by binding of sendai virus to lactosylceramide, GlcNAcβ3Galβ4GlcβlCer, GalNAcβ4Galβ4GlcβlCer, Galα3/4Galβ4GlcβlCer, Galβ3/4GlcNAcβ3Galβ4GlcβlCer (US patent No. 4,859,769, Karlsson et al., August 1989). US patent 5,242,800 by Jimenez et al. (September 1993) shows that lactosylceramide is receptor for pathogenic fungi. Oligosaccharide sequences Gala-, GalNAcα/β3-, or GlcNAcβ-3Galβ4GlcNAc are receptors for toxin A of *Clostridium difficile* (Teneberg et al., 1996). The saccharides GlcNAcβ3Galβ, Galβ3GlcNAc, Galβ3/4GlcNAcβ3Galβ4Glc, and Galβ4Glcβ are known as potential receptors for *Streptococcus pneumoniae* (Andersson *et al.,* 1986). An accompanying patent application (FI20010118) describes novel neutral oligosaccharide receptors for *Helicobacter pylori* which have saccharide sequence similar to the receptors of *C*. *difficile. Helicobacter pylori* is also known to bind lactosylceramide, asialo-GM1 and asialo-GM2-gangliosides. These studies do not relate to the use of glycosidase inhibitors.

Natural glycoconjugates are usually biodegradad by glycosidase enzymes. Specific glycosidases cleaving the animal and human monosaccharide residues are known. Beside animals and humans glycosidases are also present in yeasts, plants, viruses and bacteria. Glycosidases of pathogens are thought to cleave human or animal glycoconjugates to release carbohydrates for their own metabolic consumption. Numerous inhibitor molecules have described to prevent glycosidase reactions in most cases in biochemical experiments with enzymes or cells.

*Helicobacter pylori* has been implicated in several diseases present in the gastrointestinal tract or other organs including chronic gastritis, non-steroidal anti-inflammatory drug (NSAID) associated gastric disease, duodenal and gastric ulcers, gastric MALT lymphoma and gastric adenocarcinoma (Axon, 1993; Blaser, 1992; DeCross and Marshall, 1993; Dooley, 1993; Dunn *et al.,* 1997; Lin *et al.,* 1993; Nomura and Stemmermann, 1993; Parsonnet *et. al.* 1994; Sung, et al., 2000; Wotherspoon *et al.,* 1993). Totally or partially non gastrointestinal diseases include sudden infant death syndrome (Kerr *et al.,* 2000 and

US 6,083,756), autommune diseases such as autoimmune gastritis and pernicious anaemia (Appelmelk *et al.,* 1998; Chmiela et al, 1998; Clayes *et al.,* 1998; *Jassel et al.,* 1999; Steininger *et al.,* 1998) and some skin diseases (Rebora *et al.,* 1995), pancreatic disease (Correa *et al.,* 1990), liver diseases including adenocarcinoma (Nilsson *et al.,* 2000; Avenaud *et al.,* 2000) and heart diseases such as atherosclerosis (Farsak *et al.,* 2000). Multiple diseases caused or associated with *He*/*icobacter pylori* has been reviewed (Pakodi *et al.,* 2000). Of prime interest with respect to bacterial colonization and infection is the mechanism(s) by which this bacterium adheres to the epithelial cell surfaces of the gastric mucosa.

Glycoconjugates, both lipid- and protein-based, have been reported to serve as receptors for the binding of this microorganism as *e.g*. sialylated glycoconjugates (Evans *et al.,* 1988), sulfatide and GM3 (Saitoh *et al.,* 1991), Le^{b} determinants (Borén *et al.,* 1993), polyglycosylceramides (Miller-Podraza *et al.,* 1996; 1997a), lactosylceramide (Ångström *et al.,* 1998) and gangliotetraosylceramide (Lingwood *et al.,* 1992; Ångström *et al.,* 1998). Other potential receptors for *Helicobacter pylori* include the polysaccharide heparan sulphate (Ascensio *et al.,* 1993) as well as the phospholipid phosphatidylethanolamine (Lingwood *et al.,* 1992).

US patents of Zopf et al.: 5,883,079 (March 1999), 5,753,630 (May 1998) and 5,514,660 (May, 1996) describe Neu5Acα3Gal- containing compounds as inhibitors *H.pylori* adhesion. Sialyl-lactose molecule inhibits *Helicobacter pylori* binding to human gastrointestinal cell lines (Simon *et al.,* 1999) and is also effective in an rhesus monkey animal model of the infection (Mysore *et al.,* 2000). The compound is in clinical trials. The present invention is not related to use of antiadhesive oligosaccharides.

US patent Krivan et al. 5,446,681 (November 1995) describes bacterium receptor antibiotic conjugates comprising an asialo ganglioside coupled to a penicillin antibiotic.

Especially is claimed the treatment of *Helicobacter pylori* with amoxicillin-asialo-GM1 conjugate. The invention does not describe use of glycosidase inhibitors.

Several inhibitors of the sialidase (or neuraminidase) enzymes of human influenza virus, such as Zanamivir from Glaxo-Wellcome and Oseltamivir (Tamiflu) from Hoffmann-La Roche, are on the markets as drugs against influenza and a novel inhibitor RWJ-270201 from Biocryst is in late clinical trials (Gubareva *et al.,* 2000; Babu *et al.,* 2000). These are described to inhibit the propagation of the viruses by preventing the virus particles from leaving the infected cell. The other protein of influenza virus called hemagglutinin is known to bind sialylated receptors of target cells and desialylation has been only considered only to be necessary in removal of the receptors when replicated viruses are leaving a cell. Some neutral virus receptors have been described (US patent Karlsson et al., 4,859,769). A recent study describes infection of desialylated cells indicating also that desialylated receptors may be present on cell surfaces. The present invention describes prevention of secondary bacterial infection related to influenza by the use of sialidase inhibitors to prevent the sialidase of influenza of desialylating cell surface glycoconjugates to neutral receptor glycans of pathogenic bacteria.

The infection by influenza virus is often associated with bacterial infections and bacteria such as pneumonia-causing lung pathogens have been shown to infect especially well cells previously infected by the influenza virus (Davidson, *et al.,* 1981; Plotkowski, *et al.,* 1986). The role of sialidase was suggested but potential receptor epitopes revealed were not defined nor the use of sialidase inhibitors was suggested (Plotkowski, *et al.,* 1986). The copathogenesis is explained at least partly by the present invention. The sialidase enzymes of the influenza virus and glycosidases from the bacteria cleaves cell surface glycoconjugates to reveal the neutral receptors. Major parts of the mortality related to the influenza is actually caused by secondary bacterial infections, which are especially dangerous for older people in nursing homes. Current therapies against the complicated infections are still not effective enough, for instance, the vaccine protection (and often supply) is limited.

A glucosidase inhibitor is in clinical trials against a glycolipid storage disease by Oxford Glycosciences in England and a swainsonine analog which inhibits an α-mannosidase enzyme in Golgi are being studied as a cancer drug by Glycodesign in Canada. These glycosidase inhibitors involved in intracellular processes are not studied as inhibitors of pathogen adhesion.

### SUMMARY OF THE INVENTION

The present invention relates to the use of at least one a glycoinhibitor monosaccharide comprising a pyranose formed ring structure selected from the group GlcNAcβ, GalNAcβ, Galβ, Galα, GalNAcα, Fucα, Mana, and Neu5Acα, said monosaccharide being linked to an aglycon optionally with at least one oligosaccharide sequence or oligosaccharide sequences according to formula

[Galβy]ₚ[Hex(NAc)ᵣα/βz]ₛGalβ4Glc(NAc)ᵤ (I)

wherein p, r, s, and u are each independently 0 or 1, and y is either linkage position 3 or 4, and z is either linkage position 3 or 4, and Hex is either Gal or Glc,
so that when p is 1 and y=3, then Hex is Galβ or Glcβ and r=1, or p is 1 and y=4 then Hex is Glcβ and r=1, when p is 0 and z is 4, then Hex is Galβ and r is 1 or Hex is Gala and r is 0 for manufacture of medicament for the treatment of an infectious disease.

### BRIEF DESCRIPTION OF THE DRAWINGS

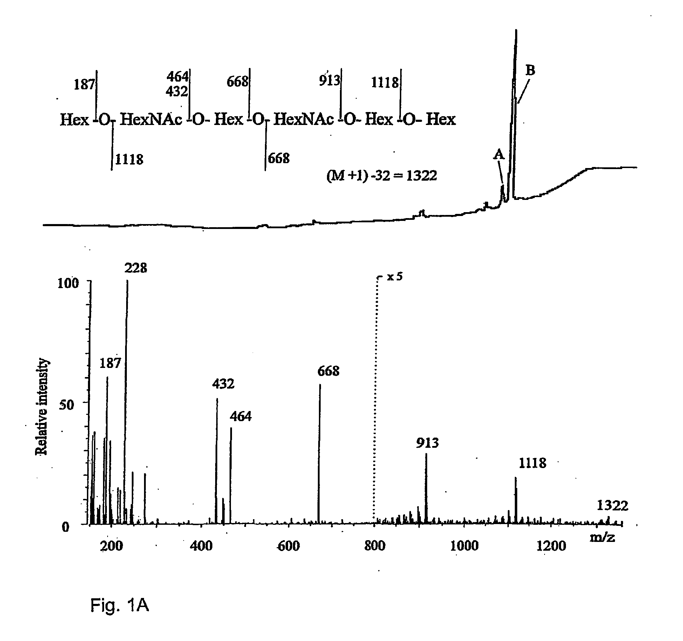

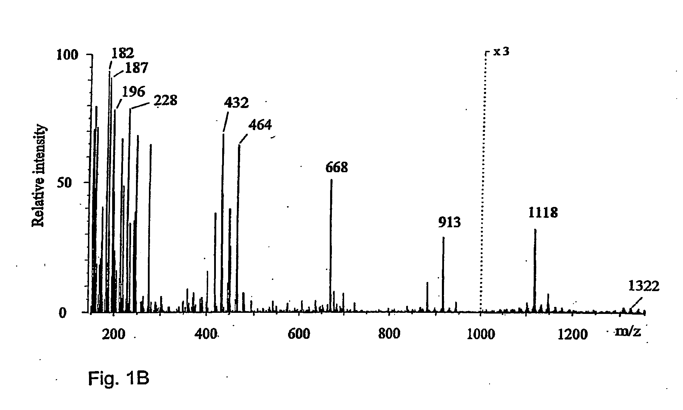

**Figs. 1A** and **1B****.** EI/MS of permethylated oligosaccharides obtained from hexaglycosylceramide by endoglycoceramidase digestion. Gas chromatogram of the oligosaccharides (top) and EI/MS spectra of peaks A and B, respectively (bottom).

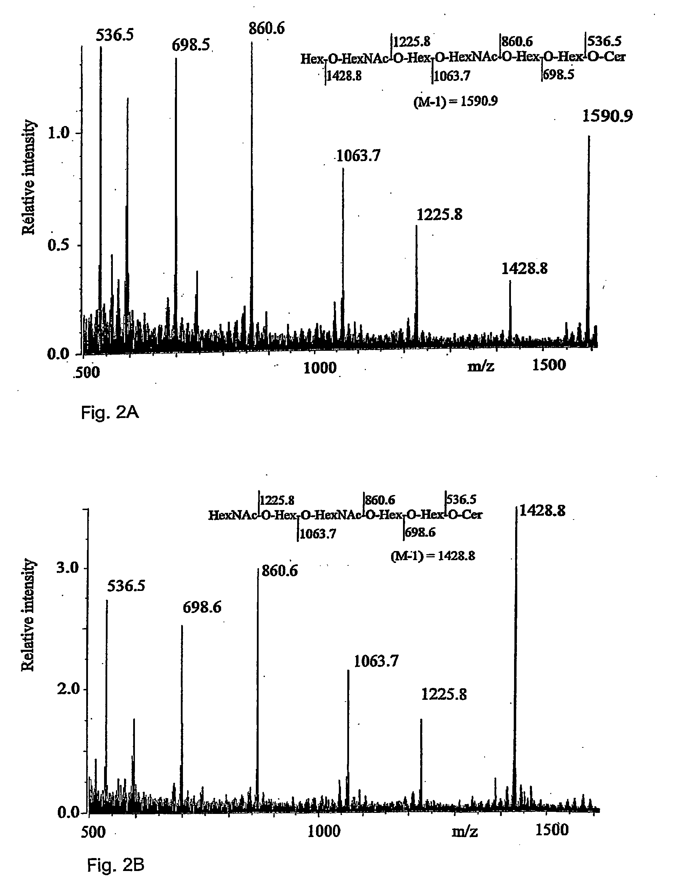

**Figs. 2A** and **2B****.** Negative-ion FAB mass spectra of hexa- **(2A)** and pentaglycosylceramide **(2B).**

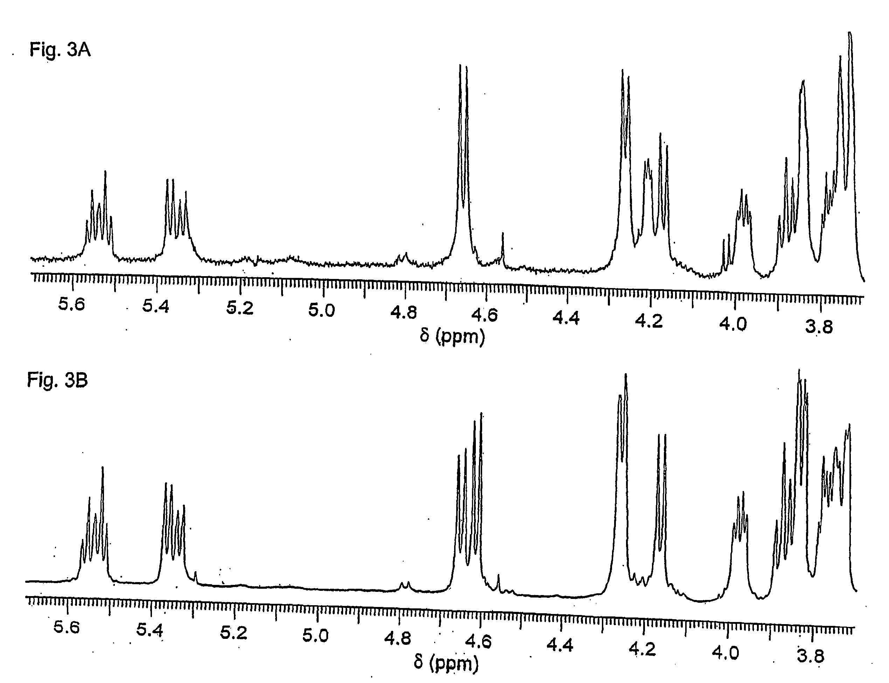

**Figs. 3A** and **3B****.** Proton NMR spectra showing the anomeric region of the six-sugar glycolipid **(3A)** and the five-sugar glycolipid (3B). Spectra were acquired overnight to get good signal-to-noise for the minor type 1 component.

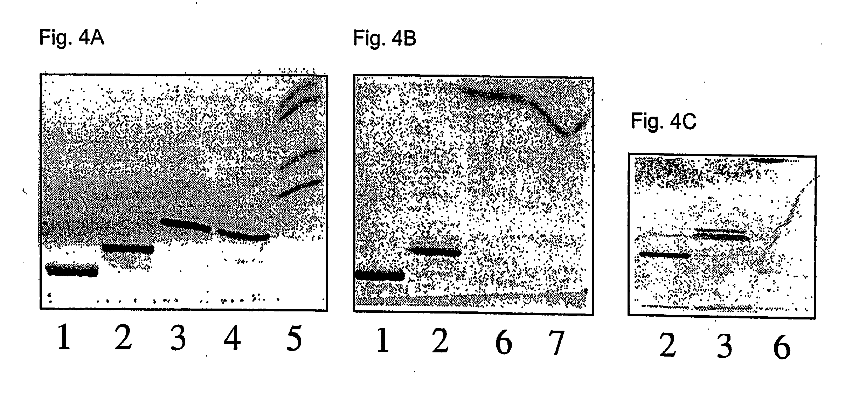

**Figs. 4A, 4B** and **4C****.** Enzymatic degradation of rabbit thymus glycosphingolipids. Silica gel thin layer plates were developed in C/M/H₂O, (60:35:8, by vol.). **4A** and **4B, 4-**methoxybenzaldehyde visualized plates. **4C,** autoradiogram after overlay with ³⁵S-labeled *Helicobacter pylori.* 1, heptaglycosylceramide (structure 1, Table I); 2, desialylated heptaglycosylceramide (obtained after acid treatmet); 3, desialylated heptaglycosylceramide treated with β4-galactosidase; 4, heptaglycosylceramide treated with sialidase and β4galactosidase; 5, reference glycosphingolipids from human erythrocytes (lactosylceramide, trihexosylceramide and globoside); 6, desialylated heptaglycosylceramide treated with β4-galactosidase and β3-hexosaminidase; 7, heptaglycosylceramide treated with sialidase, β4-galactosidase and β-hexosaminidase.

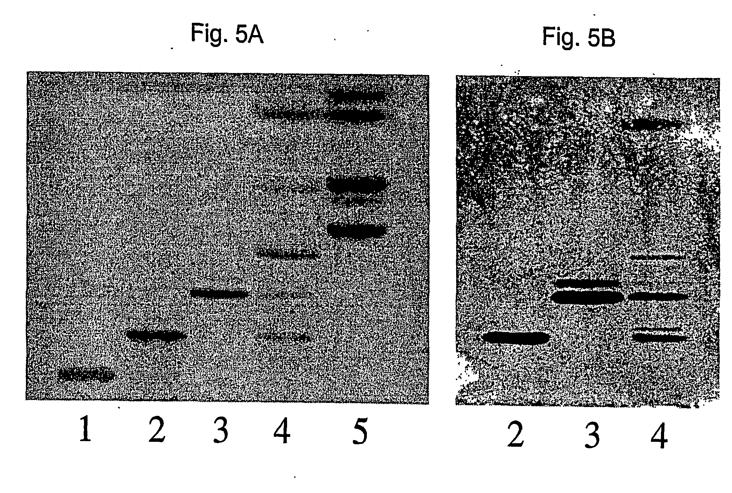

**Figs. 5A** and **5B****.** TLC of products obtained after partial acid hydrolysis of rabbit thymus heptaglycosylceramide (structure 1, Table I). Developing solvent was as for Fig. **4A**, **4B** and **4C****. 5A,** 4-methoxybenzaldehyde-visualized plate; **5B,** autoradiogram after overlay with ³⁵S-labeled *Helicobacter pylori.* 1, heptaglycosylceramide; 2, desialylated heptaglycosylceramide (acid treatment); 3, pentaglycosylceramide; 4, hydrolysate; 5, reference glycosphingolipids (as for Figs. 4A, 4B and 4C).

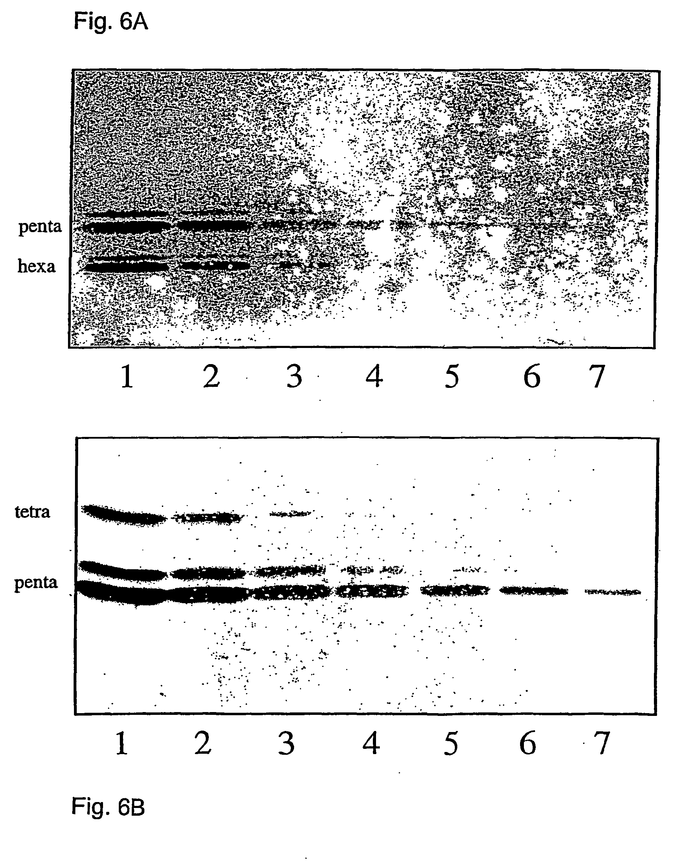

Figs. **6A** and **6B**. Dilution series of glycosphingolipids. The binding activity on TLC plates was determined using bacterial overlay technique. TLC developing solvent was as for Figs. 4A, 4B and 4C. Different glycolipids were applied to the plates in equimolar amounts. Quantification of the glycolipids was based on hexose content. **6A,** hexa- and pentaglycosylceramides (structures 2 and 3, Table I); **6B,** penta- and tetraglycosylceramides (structures 4 and 5, Table I). The amounts of glycolipids (expressed as pools) were as follows: 1, 1280 (of each); 2, 640; 3, 320; 4, 160; 5, 80; 6, 40; 7, 20 pmols (of each).

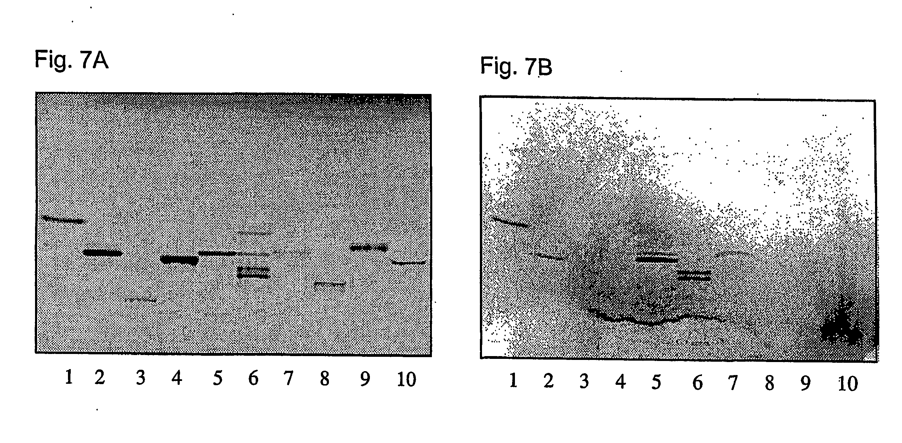

**Figs. 7A** and **7B****.** Thin-layer chromatogram with separated glycosphingolipids detected with 4-methoxybenzaldehyde **(7A)** and autoradiogram after binding of radiolabeled *Helicobacter pylori* strain 032 **(7B).** The glycosphingolipids were separated on aluminum-backed silica gel 60 HPTLC plates (Merck) using chloroform/methanol/water 60:35:8 (by volume) as solvent system. The binding assay was done as described in the "Materials and methods" section. Autoradiography was for 72 h. The lanes contained:
   lane 1) Galβ4GlcNAcβ3Galβ4Glcβ1Cer (neolactotetraosylceramide), 4 µg;
   lane 2) Galα3Galβ4GlcNAcβ3Galβ4Glcβ1Cer (B5 glycosphingolipid), 4 µg;
   lane 3) Galα3Galβ4GlcNH₂β3Galβ4Glcβ1Cer, 4 µg;
   lane 4) Galα3(Fucα2)Galβ4GlcNAcβ3Galβ4Glcβ1Cer (B6 type 2 glycosphingolipid), 4 µg;
   lane 5) GlcNAcβ3Galβ4GlcNAcβ3Galβ4Glcβ1Cer, 4 µg;
   lane 6) Galβ4GlcNAcβ3Galβ4GlcNAcβ3Galβ4Glcβ1Cer, 4 µg;
   lane 7) GalNAcβ3Galβ4GlcNAcβ3Galβ4Glcβ1Cer (x₂ glycosphingolipid), 4 µg;
   lane 8) NeuAcα3GalNAcβ3Galβ4GlcNAcβ3Galβ4Glcβ1Cer (NeuAc-x₂), 4 µg;
   lane 9) Fucα2Galβ4GlcNAcβ3Galβ4Glcβ1Cer (H5 type 2 glycosphingolipid), 4 µg;
   lane 10) NeuAcα3Galβ4GlcNAcβ3Galβ4Glcβ1Cer (sialylneolactotetraosylceramide), 4 µg. The sources of the glycosphingolipids are the same as given in Table 2.

### DETAILED DESCRIPTION OF THE INVENTION

Present invention relates to neutral non-sialylated oligosaccharide sequences of animal and human cells which can serve as receptors for numerous bacteria and viruses. Many, if not most or all, pathogens bind to specific oligosaccharide sequences on animal cell surfaces. Evolutionary pressure by the pathogen adhesion drives animal cells to change the cell surface glycosylations, especially the terminal structures to prevent such bindings. The present invention teaches that pathogenic agents may use specific glycosidase enzymes to remove protecting modification to create more common oligosaccharide receptor glycan epitopes. The present invention is especially directed to inhibition of soluble protein type pathogenic agents. The soluble protein type pathogenic agents are preferably glycosidase enzymes which can degrade human epithelial carbohydrates to receptors of pathogens. In another preferred embodiment the soluble pathogenic agents are plant lectins binding human tissues and causing harmful effects including toxic effect, the plant lectins are specifically toxic plant lectins. In third preferred embodiment the soluble protein type pathogenic agent is a toxic lectin from non-plant origin, preferably a toxic lectin or protein toxin from bacteria or toxic lectin of animal origin. The pathogenic agents can also be fungi, viruses, or bacteria, these microbial pathogenic agents are inhibited by inhibition of the glycosidase inhibitors or specific glycoinhibitors according to the present invention or also by direct inhibition pathogen adhesion. The present invention is directed to specific *in vivo* and *ex vivo* uses. The invention describes use of glycosidase inhibitors to prevent synthesis of the neutral glycan receptors for the pathogenic agents and thereby treatment of diseases and conditions due to the pathologic process.

*Receptors of pathogenic agents.* A common characteristic to the receptors of the pathogenic agents is that the receptors are neutral and can be formed by degradation of human or animal glycoconjugates by glycosidases. The neutral receptors are present in core regions of glycosphingolipids, in the backbone of common poly-N-acetyllactosamines or in defucosylated forms of blood group A or B antigens. It is considered beneficial for a pathogen to target its binding to conserved parts of glycosylation. Many glycoconjugates contain acidic modifications by sialic acids or are fucosylated, these modifications are more terminal and varying, possibly due to evolutionary pressure by the pathogens, such acidic saccharide sequences or fucosylated sequences and specific pathogen binding mechanisms to these are not in the scope of the present invention. The fucose and sialic acid decoys are probably aimed by evolution to protect the core neutral glycan epitopes from binding of pathogenic agents, presence of glycosidase with pathogenic agents may break this protection. The common structural feature to the receptors is presence of lactose (Galβ1-4Glc) and/or type 2 N-acetyllactosamine (Galβ1-4GlcNAc) as major binding epitopes, these two sequences can be modified by mono- or oligosaccharides at position 3 or 4 of galactose residue depending on the fine specificity of the binding. In some cases variants of the receptor have conformationally analogous type 1 N-asetyllactosamine (Galβ1-3GlcNAc) as major binding structure.

Lactocylceramide is also a common receptor for pathogenic bacteria and viruses. A thorough early review of pathogen binding to glycans shows that binding to lactosyl sequence is common to numerous mainly pathogenic bacteria (several of genus *Bacteroides, Clostridium, Shigella, Fusobacterium, Yersinia,* and *Salmonella typhimurium, E. coli, Campylobacter jejuni, Haemophilus influenzae, Pseudomonas aeruginosa, Vibrio cholerae,* and *Neisseria gonorrheae*) and certain toxins. The longer binding epitopes (described in more detail below) with substituted lactose-residues can be considered as specific modifications of lactosyl (Galβ4Glcβ) which are tolerated by the specificities of the pathogenic agents (Karlsson, 1989). The newer specificities towards N-acetyllactosamine (Galβ4GlcNAcβ) sequences can be recognized as a variation of the binding to very backbone sequences of glycan.
Lactosylceramide has also been shown to bind cell surface polysaccharide present on pathogenic yeasts (alfa-beta tech. inc., USA). US patent by Jimenez et al., 5,242,800 (September 1993) shows that lactosylceramide is receptor for pathogenic fungi. Saccharide sequence Galα4Galβ4Glcβ is a receptor of shiga toxins of *Shigella dysenteriae,* shiga-like toxins (verotoxins) of *Escherichia coli* and uropathogenic *E. coli.* The toxins cause diarrhea with bleeding and the saccharide is currently in phase 3 clinical trials against HUS, (hemolytic uremic syndrome, Synsorb inc., Canada) while uropathogenic *E. coli* causes urinary tract infections.
Several neutral and relatively rare oligosaccharide sequences are present on animal and human glycoconjugates. The saccharide sequences of asialo-GM1 (Galβ3GalNAcβ4Galβ4GlcβCer) and asialo-GM2 (GalNAcβ4Galβ4GlcβCer) glycosphingolipids are known receptors for numerous pathogenic agents including many pathogenic bacteria present in lungs, and viruses such as rotavirus. US patents by Krivan, et al. 5,386,027 (January 1995) and 5,217,715 (June 1993) describe use of asialo-GM1 and asialo-GM2 to inhibit several pathogenic bacteria.
Several viruses including human influenza virus, rotavirus, reovirus, mumps virus and rabies virus bind to glycolipids containing an oligosacharide sequence up to tetrasaccharide as exemplified by binding of sendai virus to lactosylceramide, GlcNAcβ3Galβ4Glcβ1Cer, GalNAcβ4Galβ4Glcβ1Cer, Galα3/4Galβ4Glcβ1Cer, Galβ3/4GlcNAcβ3GalβGlcβ1Cer (US patent, Karlsson et al., 4,859,769, August 1989). Oligosaccharide sequences Gala-, GalNAcαorβ-, or GlcNAcβ-3Galβ4GlcNAc are receptors for toxin A from *Clostridium difficile* (Teneberg et al., 1996). The saccharides GlcNAcβ3Galβ, Galβ3GlcNAc, Galβ3/4GlcNAcβ3Galβ4Glc, and Galβ4Glcβ are known as potential receptors for *Streptococcus pneumoniae* (Andersson *et al.,* 1986). An accompanying patent application (FI20010118) describes novel neutral oligosaccharide receptors for *Helicobacter pylori* which have saccharide sequences similar to the receptors of *C. difficile.*

The present invention relates also to a family of specific novel oligosaccharide sequences binding to *Helicobacter pylori.* These novel neutral oligosaccharide receptors, their analogs and use thereof are described in a co-pending application: Novel receptors for *Helicobacter pylori* and use thereof (FI20010118). The natural types of the receptors described including GlcNAcβ3Galβ4GlcNAc, GalNAcβ3Galβ4GlcNAc, GalNAcα03Galβ4GlcNAc, Galβ3Galβ4GlcNAc, Galα3Galβ4GlcNAc are especially of interest of present study as receptors made by degradation of larger oligosaccharide structures.

The receptors were characterized as glycolipids with sequences
Galβ4GlcNAcβ3GalβGlcβer, Galβ4GlcNAcβ3Galβ4GlcNAcβ3Galβ4GlcβCer, GalNAcβ3Galβ4GlcNAcβ3Galβ4GlcβCer, GalNAcα3Galβ4GlcNAcβ3Galβ4GlcβCer, GlcNAcβ3Galβ4GlcNAcβ3Galβ4GlcβCer and Galα3Galβ4GlcNAcβGalβ4GlcβCer and Galα3Galβ4GlcNAcβ3Galβ4GlcNAcβ3Galβ4GlcβCer, but similar oligosaccharide sequences are also present on glycoproteins. The binding epitope was shown to include the terminal trisaccharide element of active pentasaccharide glycolipids, at least in larger repetitive N-acetyllactosamines the epitope may also be in the middle of the saccharide chain. The inventors realize that the binding epitopes can be presented in numerous ways on natural or biosynthetically produced glycoconjugates and oligosaccharide such as ones of O-linked or N-linked glycans of glycoproteins and on poly-N-acetyllactosamine oligosaccharides. The similarity of the epitopes was shown by molecular modelling of the glycolipids.

*Glycosidases.* The term "glycosidase" refers here to enzymes capable of cleaving monosaccharides (from the terminal non-reducing end, i.e. exoglycosidases) or oligosaccharides (endoglycosidases) from oligosaccharide chains and/or saccharide conjugates, where the released saccharide can be modified for example by a double bond. The monosaccharide to be cleaved from the glycoconjugate of the patient is preferably sialic acid (neuraminic acid), especially D-Neu5Ac or D-Neu5Gc, L-Fuc, D-Gal, D-GlcNAc, D-GalNAc, more preferably α-D-Neu5Ac, α-L-Fuc, β-D-Gal, β-D-GlcNAc, most preferably the said Neu5Ac is α3-, α8- or α6-linked [Neu5Acα(2-3)- etc] to the next monosaccharide residue. Fuc is α2-, α3- or α4-linked, Gal is β3- or β4-linked, and GlcNAc is β3- or β6-linked. The exo-glycosidase enzyme are preferably sialidase or N-acetylneuraminidase, α-fucosidase, β-galactosidase, β-N-acetylhexosaminidase or β-N-acetylglucosaminidase capable of cleaving monosaccharides with one or more of the linkages described above. The endo-glycosidase enzyme cleaves preferably poly-N-acetyllactosamines such as endo-β-galactosidase or endo-β-N-acetylglucosaminidase. The neutral receptor epitopes can also be revealed by sulphatase enzymes from sulphated glycoconjugates and such a process could be inhibited by inhibitors of sulphatase enzymes.

*Glycosidase inhibitors.* Glycosidase inhibitors for an exoglycosidases are usually analogs of the terminal monosaccharide to be cleaved by an exoglycosidase enzyme. The analog may mimic the transition state of the glycosidase reaction, which may give strong binding to enzyme, for example such as nojirimycin and lactone analogs of monosaccharides. Free monosaccharides and even oligosaccharides and derivatives containing the same terminal monosaccharide with the same anomeric structure (α or β, free reducing monosaccharides have usually both in water solutions), which is cleaved by the glycosidase enzyme, can also be used as competive inhibitors, but large concentrations in range from millimolar up to several hundred millimolar may be needed. Oligosaccharides may be more effective than monosaccharides. When a monosaccharide is used according to the invention, it is not used as antiadhesive compound to block a lectin or lectin-like receptor, but as an inhibitor to prevent glycosidase enzymes to form neutral oligosaccharide receptors for pathogenic agents. The inhibitor may also be a monosaccharide derivative in which the glycosidic linkage is not cleaved by glycosidases, such as analogs where the glycosidic oxygen atom is replaced by sulphur (thioglycosidic bond) or carbon (C-glycosidic bond) atoms. Oligosaccharides containing such analogs of glycosidic bond are also glycosidase inhibitors. An endoglycosidase inhibitor can be made by having such unhydrolyzable bond in the middle of an oligosaccharide in position to be cleaved by an endoglycosidase, for example Galβ4GlcNAcβ3Galβ-S-4Glc (-S- is a tioglycosidic bond in place of O-glycosidic linkage) is potential inhibitor for endo-β-gactosidase type enzymes.

Examples of glycosidase inhibitors includes 2,3-anhydro-2-deoxy-N-acetylneuraminic acid (Neu5Ac2en, DANA; reference: Kopitz *et al*.1997), para-aminophenyl-β-D-thiogalactopyranose (McGee and Murray,1986), N-(n-butyl)-deoxygalactonojirimycin (Platt *et al.,* 1994), deoxygalactonojirimycin, deoxyfuconojirimycin (1,5-dideoxy-1,5-imino-L-fucitol, Winchester *et al.* 1990), which are available for example from Calbiochem, La Jolla, USA; 2-acetamido-1,2-dideoxynojirimycin (Woynamwska *et al.,* 1992) and 2-acetamido-2-deoxy-D-glucono-1,5-lactone (Sasaki *et al.,* 1997), which are available from Toronto Research Chemicals, North York, Alberta, Canada; D-galactonic acid gamma-lactone (D-galactono-1,4-lactone; Huber and Brockbank, 1987, actual active form is probably D-galactono-1,5-lactone) which is available from Sigma, St Louis, USA. More glycosidase inhibitors and their synthesis has been described in US patent No. 5,461,143 by C.-H. Wong. Various glycosidase inhibitors and uses thereof has also been described e.g. in the following articles and in the references cited therein (Huber and Brockbank, 1987; Kumar et al.,1981; Nöhle et al., 1985; Schreiner et al., 1985). Medical use of sialidase inhibitors as novel anti-fluenza drugs has been reviewed (Gubareva *et al.,* 2000; Babu *et al.,* 2000).

*Target cells.* Target cells for *Helicobacter pylori* are primarily epithelial cells of the target tissue especially in the gastrointestinal tract, other potential target tissues are for example liver and pancreas. Glycosylation of the target tissue may change due to an infection by a pathogen. Target cells may also be malignant, transformed or cancer/tumour cells in the target tissue. Transformed cells and tissues express altered types of glycosylation and may provide receptors for bacteria. Binding of lectins or saccharides (carbohydrate-carbohydrate interaction) to saccharides on glycoprotein or glycolipid receptors can activate cells, in case of cancer/malignant cells this may be lead to growth or metastasis of cancer. Several of the oligosaccharide epitopes of the invention, such as GlcNAcβ3Galβ4GlcNAc (Hu, J. *et al.,* 1994), Galα3Galβ4GlcNAc (Castronovo *et al.,* 1989), and neutral and sialylated polylactosamines (Stroud *et al.,* 1996) from malignant cells have been reported to be cancer-associated or cancer antigens. *Helicobacter pylori* is associated with gastric lymphoma. The substances according to the invention can be used to prevent binding of *Helicobacter pylori* to premalignant or malignant cells and activation of cancer development or metastasis. Inhibition of the binding may cure gastric cancer, especially lymphoma.

Target cells also include blood cells, especially leukocytes. It is known that *Helicobacter pylori* strains associated with peptic ulcer, as the strain mainly used here, stimulates an inflammatory response from granulocytes, even when the bacteria are nonopsonized (Rautelin *et al.,* 1994a,b). The initial event in the phagocytosis of the bacterium most likely involves specific lectin-like interactions resulting in the agglutination of the granulocytes (Ofek and Sharon, 1988). Subsequent to the phagocytotic event oxidative burst reactions occur which may be of consequence for the pathogenesis of *Helicobacter pylori*-associated diseases (Babior, 1978).

*Specific control of pathogenic strains of microbes.* A specific aim of the invention is to prevent or inhibit subpopulations of viruses, toxins, bacteria or fungi which bind the pathogenesis-related neutral oligosaccharide receptors. Such subpopulations are specifically pathogenic agents, while other forms of same viruses, toxins, bacteria or fungi do not bind the neutral receptors and are generally less pathogenic or non-pathogenic at least for the major normal population which has the receptor covered by substituting residues during normal conditions. The current therapies with antibiotics remove totally multiple microbial species and strains which include also harmless and beneficial species. Most of the microbes of the so called normal flora are necessary symbionts of humans (or animals) facilitating food digestion and producing important cofactors and vitamins and preventing adhesion or propagation of severe pathogens. Destruction of total population of beneficial bacteria is therefore a major drawback of current chemotherapies by regular antibiotics or unspecific vaccinations. The present innovation aims at control of pathogenic strains of microbes.

It is known that *Helicobacter pylori* can bind several kinds of oligosaccharide sequences. Some specificities of certain strains represent symbiotic interactions which do not lead to cancer or other severe conditions. The present data about binding to cancer-type saccharide epitopes indicates that the substance according to the invention can prevent pathologic interactions, and in doing this, it may not effect on some of the less pathogenic *Helicobacter pylori* bacteria/strains binding to other receptor sructures. Therefore total removal of the bacteria may not be necessary for the prevention of the diseases realated to *Helicobacter pylori.* The less pathogenic bacteria may even have a probiotic effect as they can prevent the colonization of the pathogenic strains of *Helicobacter pylori* in the gastric tract.

According to the invention it is possible to incorporate the substance according to the invention, optionally with a carrier, in a pharmaceutical composition, which is suitable for the treatment of a condition due to the presence of *Helicobacter pylori* in the gastrointestinal tract of a patient or to use the substance according to the invention in a method for the treatment of such conditions. Examples of conditions treatable according to the invention are chronic superficial gastritis, gastric ulcer, duodenal ulcer, non-Hodgkin lymphoma in human stomach, gastric adenocarcinoma, certain pancreatic, skin, liver, or heart diseases, sudden infant death syndrome, autoimmune diseases including autoimmune gastritis and pernicious anaemia and non-steroid anti-inflammatory drug (NSAID) related gastric disease caused by the *Helicobacter pylori.*

The present invention is especially aimed at prevention of autoimmune diseases such as gastritis and pernicious anemia caused by *Helicobacter pylori.* An autoimmune process destroys specific gastric epithelial cells called parietal cells, which has been suggested to be associated with *Helicobacter pylori* infections by molecular mimicry, a theory which has been recently taken back by the author. Here we notice that the novel polylactosamine receptors described by the inventors are likely to be formed from sialylated and/or fucosylated oligosaccharide polylactosamines of human parietal cells (also other species specifically express polylactosamines on parietal cells) if the cells are in contact with hydrolytic agents such as glycosidase enzymes cleaving fucose and/or sialic acids. The direct binding of *Helicobacter pylori* allows modification and proteolysis of the parietal cell membrane proteins, especially the proton pump, by enzymes of *Helicobacter pylori,* which creates potential autoantigen epitopes. Autoantigenic epitopes include peptides similar to parts of *Helicobacter pylori* proteins. The present invention shows a method to prevent specifically the diseases by preventing the formation of the receptor on the gastric parietal cells. The autoimmune conditions may also lead to pathologic changes of gastric epithelium and even to formation of cancer.

The pharmaceutical composition described by the invention may also comprise other substances, such as an inert vehicle, or pharmaceutically acceptable carriers, preservatives etc, which are well known to persons skilled in the art.

The substance or pharmaceutical composition described by the invention may be administered in any suitable way, although an oral administration is preferred.

The term "treatment" used herein relates to both treatment in order to cure or alleviate a disease or a condition, and to treatment in order to prevent the development of a disease or a condition. The treatment may be either performed in a acute or in a chronic way. The glycosidase inhibitors can be administered together with other drugs such as known antibiotics used against the bacteria, virus, or fungus being the pathogenic agent Gastric pH-regulating drugs include special proton pump inhibitors such as Omeprazole, Esomeprazole, Lansoprazole and other pH regulating drugs such as ranitidin *etc.*

It is preferred to use a glycosidase inhibitor together with receptor oligosaccharides or their analogs or derivatives. It is more preferred to use a glycosidase inhibitor together with neutral oligosaccharide receptors described above and/or derivatives or analogs thereof. Such use prevent the formation of the active oligosaccharide receptors on the target cells and prevents binding to residual glycoforms containing the receptor active sequence.

It is noted that the pathogenic mechanism and treatments described here apply to animals including domestic and pet animals, most preferably mammals. However, there are often differencies in tissue specific glycosylations, infecting pathogen strains and species when compared to humans. The term "patient", as used herein, relates to any human or non-human mammal in need of treatment according to the invention.

The infection by influenza virus is often associated with bacterial infections and bacteria such as pneumonia causing lung pathogens, have been shown to infect especially well cells previously infected by the influenza virus (Davidson, *et al.,* 1981; Plotkowski, *et al.,* 1986). The role of a sialidase was suggested but potential revealed receptor epitopes were not definied nor the use of sialidase inhibitors was suggested (Plotkowski, *et al.,* 1986). The copathogenesis of influenza virus and a lung pathogen is explained at least partly by the present invention. The sialidase enzymes of the influenza virus cleaves cell surface glycoconjugates to reveal the neutral receptors for other pathogenic agents. A similar mechanism has been suggested in US patents Nos. 5,386,027 and 5,217,715 by Krivan, et al. describing the use of asialo-GM1 and asialo-GM2 to inhibit several pathogenic bacteria. The patent applications also suggest the pathogenic mechanism where desialylation by influenza virus creates the unsubstituted receptor GalNAcβ4Gal. However, in such a structure GalNAc is not known to be sialylated and when Gal is α3-sialylated the structure is resistant to most sialidases by steric hindrance. Desialylation of other structures such as the terminal Gal of GMlb is more likely to happen.

Furthermore, it is possible to use the substance according to the invention as part of a nutritional compositon, for example in food or beverage composition. It is preferred to use the substance according to invention as a part of a so called functional or functionalized food. The said functional food has a positive effect on the person's or animal's health by inhibiting or preventing the binding of *Helicobacter pylori* to target cells or tissues. The substance according to the invention can be a part of defined food or functional food composition. The functional food can contain other known food ingredients accepted by authorities controlling food such as Food and Drug Administration in USA. The substance according to invention can be also used as food additive, preferably as a food additive to produce a functional food. The origin of the glycosidase in gastrointestinal tract may be food, for example almonds and many beans contain exo-and endo-glycosidases. Adding a glycosidase inhibitor, preferably specific for the glycosidase in food, to such food reduces its capacity to create receptors for pathogens and improves its quality. Preferably natural forms of glycosidase inhibitors are used such as galactonolactones.

It is especially useful to have the substance according to the invention as part of a food for an infant or child, preferably as a part of an infant formula. Many infants are fed by special infant formulas as replacement of natural human milk. *Helicobacter pylori* is especially infective to infants or young children, considering the diseases it may later cause it is reasonable to prevent the infection. *Helicobacter pylori* is also known to cause sudden infant death syndrome, but the strong antiobiotic treatments used to eradictate the bacterium may be especially unsuitable for young children or infants.

The invention is also directed to the use of the oligosaccharide sequences according to the invention in food safety products for inhibition of pathogenic agents, glycosidases especially in connection with bacteria such as *Helicobacter pylori* or diarrheagenic *E. coli.* The present invention is also directed to food safety methods to inhibit pathogens, harmful lectins and glycosidases.

### Glycoinhibitors

Glycoinhibitors include traditional glycosidase inhibitors as described by the invention. The glycoinhibitors are especially aimed for inhibition of glycosidase enzymes human or animal gastrointestinal tract The glyosidases targeted are especially exoglycosidases chosen from the group: β-N-acetylhexosaminidases, β-N-acetylglucosaminidases, β**-**N-acetylgalactosaminidases, β-galactosidases, β-xylosidases, α-galactosidases, α-N-acetylgalactosaminidases, β-N-acetylglucosaminidases, α-fucosidases, α-mannosidases and α-N-acetylneuraminidases. The glycoinhibitor-type glycosidase inhibitors are preferably aimed for use in polyvalent forms described by the invention glycosidase inhibitors The glycoinhibitors are especially aimed as glycosidase inhibitors for prevention of pathogenesis by bacteria by inhibition of glycosidases revealing the receptor for the pathogens. The glycoinhibitors are specifically aimed for theraphy of animals and humans

In a separate embodiments the glycoinhibitors are aimed for inhibition of harmful lectins in food stuffs. In another separate embodiment the present invention is targeted for inhibition of food glycosidases cleaving carbohydrates from foods. The glycoinhibitors preventing the release of carbohydrates from foods are targeted to reduce calorie content of foods. All the glycosidases described can degrade corresponding carbohydrate structures from animal based and from some plant or even yeast based foods.. For this use of lowering the calorie content the inhibitors of amylase type enzymes are and other α-glucose cleaving enzymes, which can prevent the break down of starch and/glycogen from food and thus preferred. The invention is also specifically targeted for the inhibition of fructose and or fructose releasing enzymes like sucrase-type enzymes cleaving sucrose.. Preferably the glycoinhibitors according to the present invention have additional activities as inhibitors of cell adhesion of the pathogens targeted by the glycoinhibitors. The glycoinhibitors inhibit cell adhesion mediated by glycosidase enzymes on cell surfaces, moreover the glycoinhibitors may bind to cell adhesion lectins or adhesins for inhibition of the binding of pathogenic bacteria or other pathogenic agents. The glycoinhibitors are in a preferred embodiment polyvalent conjugates. The polyvalent conjugates are in a preferred embodiment soluble polyvalent conjugates of the glycoinhibitors. The soluble polyvalent conjugates according to the present invention can bind cell surface glycosidase or lectin/adhesin on the pathogenic agent preferably a bacterium and block sterically other binding interactions of the pathogenic agents.

### Use of non-reducing pyranose formed monosaccharide residues and monosaccharidemimetics

The glycoinhibitors are non-reducing pyranose formed monosaccharide residues having the structures chosen from the group of following glycoinhibitor types: GlcNAcβ, GalNAcβ, Galβ, Gala, GalNAcα, Fucα, Manα, Xylβ, and Neu5Acα, said monosaccharide being linked to an aglycon. The monosaccharides function as competive inhibitors of glycosidase enzymes and/or inhibitors of lectins or adhesins.

### Monosaccharide mimetics

Monosaccharide mimetics according to the present inhibitors comprises traditional monosaccharide analogs used as glycosidase inhibitors. The mimetic structures have in general higher affinities for glycosidases than the monosaccharide residues. Numerous structural principles in makind analogs are known in the art. The monosaccharide mimetic comprises a structure which can be recognized by a plant or bacterial glycosidase enzyme. The monosaccharide mimetics preferably comprises modified anomeric carbon structure. The modified anomeric carbon structure preferably comprise a double bond structure like in Neu5Ac-2en, a N-acetylneuraminic acid analog which contains double bond between C2 and C3 or lactones when C1 position is in carboxylic acid form. The double bond structures are aimed for making glycosidase transition state analog-inhibitors. In a preferred embodiment the analog comprise 1-deoxy structures like in deoxynojirimycin type inhibitors such as deoxy-mannonojirimycin, deoxygalactonojirimycin, deoxygluconojirimycin. The third preferred type of modified monosaccharide mimetic is directed to monosaccharide analogs having carbon (C-glycoside), nitrogen (N-glycoside), or sulphur (S-glycoside) atom in glycosidic linkage in the place of oxygen in normal glycosidic bonds. Preferably the glycosidic bond is replaced by C-glycosidic or N-glycosidic bond and most preferably N-glycosidic bond.
Due to similarity between monosaccharides preferred cheap monosaccharide to be used as analogs includes Glcα, Glcβ, Xylα, Fruβ and Fruα, more preferably Glcα and Glcβ, which mimics in many recognitions Manα-structures and GlcNAcβ-structures, espectively, and Fruα for axial dihydroxyl recognitions is also preferred. The glycoinhibitors which can inhibit fructose and glucose release are also especially preferred for inhibition of energy release from food suffs.

### Monosaccharide glycoinhibitors

When the glycoinhibitor is larger than a monosaccharide, the non-reducing pyranose formed monosaccharide residue is linked to an aglycon mimicking next monosaccharide in potential natural receptor which participates in recognition to be inhibited. Aglycon means that the glycoinhibitor is not linked to another monosaccharide by a natural O-glycosidic bond. In a specific embodiment the aglycon is a non monosaccharide spacer linking the glycoinhibitor to a carrier, preferably to a polysaccharide or oligosaccharide carrier. The monosaccharide residues are preferably linked from C1 of the monosaccharide residue, other linkage positions especially for polyvalent conjugates can be used, primary hydroxyl groups like C6-structures in hexosopyranoses can be derivatized by oxidation, for example to aldehydes, for further derivatization. Modification acetemido groups to spacer amide groups ofN-acetyl-function comprising monosaccharidesis possible forming a type of mimetic. More preferably the aglycon structure is polyhydroxyl substance partially mimicking next monosaccharide of the corresponding oligosaccharide sequence. In a preferred embodiment the polyhydroxyl susbtance is a non-carbohydrate substance and most preferreably the polyhydroxyl substance is a flexible hydrophilic linker described by Formula 2 in this invention. Glycoinhibitors include polyvalent conjugates and soluble polyvalent conjugates of the partial oligosaccharide sequences as described elsewhere in the invention. The monosaccharide mimetics can be linked from C1 of the monosaccharide residue, other linkage positions especially for polyvalent conjugates can be used, primary hydroxyl groups like C6-structures in hexosopyranose analogous structures can be derivatized by oxidation, for example to aldehydes for further derivatization. Modification acetemido groups to spacer amide groups of N-acetyl-function comprising monosaccharide mimetic.

In a preferred embodiment the glycoinhibitor is chosen from the group GlcNAcβ, Galβ, Galα, Fucα, or Manα. In a more preferred embodiment the glycoinhibitor is chosen from the group GlcNAcβ, Galβ, Fucα, or Manα, in another preferred embodiment from the group GlcNAcβ, Fucα, or Manα. The partial oligosacharide sequences are preferably used together with low cost oligosaccharide sequences. Preferably glycoinhibitor in pyranose form is used together with at least one, and preferably with two oligosaccharide sequences, and most preferably with three oligosaccharide sequences, according to the present invention. In another embodiment at least two glycoinhibitor sequences are used with at least one oligosaccharide sequence according to the present invention. The glycoinhibitors are preferred for therapheutic uses according to the present invention, especially for feed and food uses.

### Inhibition of pathogenic agents by monovalent glycoinhibitors

An approach using glycoinhibitors could save costs of synthesis. The effect is in general based on the high concentrations used when a lectin or adhesin recognizing longer oligosaccharide sequences is inhibited. As monosaccharides are in general much cheaper than oligosaccharides the present invention is especially directed to inhibition of cell adhesion when a pathogen cell is binding to patients tissue by a glycosidase enzyme mediating cell adhesion.

The monovalent glycoinhibitors here are for example monovalent glycosidic conjugates of the monosaccharide, for example glycosylamines or glycosylamides or methyl glycosides or other glycosides including other N-glycosides, C-glycosides or S-glycosides, more preferably C- or N-glycosides and most preferably N-glycosides.

### Polyvalent carriers and conjugates

In a preferred embodiment the glycoinhibitors according to the present invention are linked to a polyvalent carrier, more preferentially at least two glycoinhibitors sequences. In a specific embodiment at least two different glycoinhibitor types are linked to the same polyvalent carrier. The polyvalent carrier is preferentially a carbohydrate carrier such as polysaccharide or an oligosaccharide, in a preferred embodiment the carbohydrate carrier is soluble carbohydrate carrier. The carbohydrate carrier is in a preferred embodiment a bacterial polysaccharide.

In another embodiment the pathogen inhibiting glycoinhibitor or glycoinhibitors are expressed on a particle carrier. The particle carrier is preferably a carbohydrate particle, a synthetic polymer particle or a cell. The cell is preferably a bacterial cell or a yeast cell. The preferred diameter of the particle is between 10 nm and 10 micrometers.

The polyvalent conjugates are preferentially designed to be non-antigenic, and non-immunogenic, so that the only minor immune reactions or no immune reactions at all are caused by the conjugates. Other preferred properties of the polysaccharides includes low toxicity of the polysaccharide and/or its degradation products.

The polyvalent conjugates which are aimed to inhibit bacteria are designed to avoid carbohydrates binding specificity or specificities of the epithelium when the carbohydrate binding specificity can attach the conjugate to the epithelium and increase the pathologic binding of the pathogen to the tissue.

Bacterial exopolysaccharides or capsular polysaccharides from bacteria are preferred, especially when the bacterium is a non-pathogenic bacterium such as lactic acid bacterium. Several of the glycoinhibitors according to the invention are known from bacterial polysaccharides. The invention is also directed to the engineering of the glycoinhibitors on bacterial polysaccharides, especially polysaccharides of non-pathogens such as lactic acid bacteria. The engineering may be done genetically or by chemical modification of the polysaccharides, for example by specific hydrolysis or glycosyltransferase reactions. According to present invention it is possible to use a bacterial polysaccharide or mixture of bacterial polysaccharides which comprises at least two types of glycoinhibitors according to the present invention. It is more preferred to use a bacterial polysaccharide or mixture of bacterial polysaccharides which comprises at least three types of glycoinhibitors and in another embodiment at least four types of glycoinhibitors according to the present invention.

In a preferred embodiment to the invention is directed chemically synthesised polyvalent glycoinhibitors wherein the glycoinhibitor structure is polyvalently or oligovalently conjugated preferrably through a spacer or non-oxygen atom to a carrier structure which is preferably a polysaccharide. In a preferred embodiment the glycoinhibitor is a pyranose linked with a glycoinhibitor of non-reducing pyranose formed monosaccharide residues described by the present invention. In another embodiment the present invention is directed to polyvalent homooligosaccharide glycoinhibitors wherein the glycoinhibitor structure is polyvalently or oligovalently conjugated preferrably through a spacer or non-oxygen atom to a carrier structure which is preferably a polysaccharide.

### Soluble polyvalent conjugates covering and agglutinating the pathogens

Preferred polyvalent conjugates include soluble or gel forming polyvalent conjugates. More preferably the polyvalent conjugate is soluble and can cover the surface of a pathogenic agent like harmful protein or bacterium. When targeting single proteins smaller conjugates are preferred than with bacteria. Preferrably the bacterium covering soluble polyvalent conjugate has at least a molecular weight of 5000 daltons, more preferably at least about 10 000 daltons and in separate embodiment at least about 20 000 daltons. For several applications higher molecular weights should be limited because of the effective diffusion of the conjugates in the gastric mucosa. Preferred upper limits of the polyvalent conjugates are under about 300 000 daltons, more preferably under about 150 000 daltons and most preferably under 50 000 daltons. More preferred molecular weight ranges include from about 5 000 to about 50 000 daltons, from about 10 000 daltons to 50 000 daltons and most preferably from about 20 000 daltons to about 100 000 daltons. Preferred molecular weight also depend on the structure of the backbone. The molecular weight limits indicate that about at least 70 % of the molecules are within the desired range and more preferably at least 80 % in the desired range. Polyvalent conjugates are also effective glycosidase inhibitor when glycosidase in represented polyvalently on a cell surface, or is oligomeric.

The polyvalent conjugates that can diffuse to the surface of the pathogenic agent and cover it are especially effective in prevention pathogenic agents when several types of bindings should be inhibited. The polyvalent conjugate or conjugates comprises carbohydrate corresponding to the most common binding activity on the pathogenic agent or pathogenic agents present. The covering of the surface by the polyvalent conjugate blocks sterically the other carbohydrate binding receptor or receptors on the surface of the pathogenic agent or the pathogenic agents. The steric inhibition requires suitable molecular weight, in general the molecular weight should be high enough to be able to effectively inhibit, on the other hand the molecular weight in certain applications should be low enough to allow effective diffusion of the soluble carbohydrate. Preferably at least two pathogen covering polysaccharides are used. More preferably two different receptor oligosaccharide sequences are conjugated to the same polymer.

The covering soluble polyvalent carbohydrate can bind several pathogenic agents together making an agglutinate which is removed for example with mucin secretion on lung or intestinal epithelium. Several pathogens can comprise several different species or strains of pathogenic agents or several cells or several protein pathogenic agentss of the same species, strain or type.

Most preferably the soluble polyvalent conjugate comprises a polysaccharide backbone. A preferred backbone for the soluble polyvalent conjugates is chitosan. The present invention is especially directed to synthetic glycoinhibitors substance wherein glycoinhibitor is conjugated to chitosan to form polyvalent or oligovalent conjugate, in more preferred embodiments the glycoinhibitor comprises
1. a non-reducing pyranose formed monosaccharide residue or monosaccharide residues or mimetics thereof described by the present invention. and/or
2. homooligosaccharide glycoinhibitor or glycoinhibitors according to the present invention and/or
3. heterooligosaccharide glycoinhibitor or glycoinhibitors. More preferred chitosan conjugates comprises monosaccharide residues or mimetics and/or homooligosaccharide linked to chitosan. In a preferred embodiment the chitosan conjugated glycoinhibitor is linked to chitosan through spacer according to the present invention or in a separate embodiment through a non-oxygen glycosidic linkage, most preferably N-glycosidic linkage. In a preferred embodiment at least one both monosaccharide and oligosaccharide type

### Ex vivo uses of the present invention

It is realized that the present invention can be used for inhibition of pathogenic agents including glycosidases, lectins and pathogens especially *H. pylori* or diarrhea causing *E. coli ex vivo* and that such method have use in disinfection and preservevation type applications. According to this embodiment it is preferred to use *ex vivo* the glycoinhibitors according to the present invention as part of single substances or as single substances or more preferably as composition comprising at least two types of glycoinhibitors according to the present invention for inhibition pathogenic agents such as pathogenic toxins or lectins or glycosidase even bacteria preferably diarrhea causing *E. coli..* Polyvalent conjugates according to the present invention, especially soluble polyvalent conjugates which can agglutinate pathogenic agents, preferably toxin type of lectins or diarrheagenic *E. coli,* are preferred for *ex vivo* uses. More specific glycoinhibitors targeted against diarrhea causing *E. coli* are desribed in a separate application

### Oral infections and oral health products

It is realized that infections tagetted by the present invention spread oral route, possibly also from nose to oral cavity. The present invention is directed to prevention of the infections already in human mouth. The present invention is directed to treatment of oral infections by at least two different types of glycoinhibitors which can inhibit at least two different binding specificities of pathogen, preferably orally infecting bacterium and more preferably a diarrhea causing bacterium. It is preferred to use the two types of glycoinhibitors according to the present invention as part of single substances or as single substances or as composition comprising at least two types of glycoinhibitors from different groups according to the present invention for inhibition of oral or nasal infections. According to the present invention the types of glycoinhibitors according to the present invention are used as compositions or as separate substances in products inhibiting pathogens, called here mouth hygiene products, in human mouth.

It is realized that human mouth comprises similar receptors as human intestine especially on proteins at least neolacto-receptors, mannose receptors and oligosaccharide receptors with fucose resembling glycoinhibitors according to the present invention. As a separate embodiment it is realized that the substances and compositions according to the present invention are also useful in inhibiting pathogens causing caries. In a specific embodiment the present invention is also directed to the compositions according to the present invention for treatment of other orally spreading infections like infection causing otitis media or lung infections including influenza, bronchitis or pneumonia. The mouth hygiene products according to the present invention can be also directed against caries, otitis media, bronchitis and pneumonia.

The present invention is especially directed to mouth hygiene products comprising substances or compositions comprising pathogen inhibiting glycoinhibitors, especially types of glycoinhibitors preferred according to the invention. The mouth hygiene product is preferably chosen from the group tooth pastes, mouth wash solutions, mouth tablets, chewing tablets, and chewing gums. It is preferred to use either monovalent types of glycoinhibitors or polyvalent glycoinhibitors. In another preferred embodiment the mouth hygiene product comprises polyvalent glycoinhibitors according to the present invention. Due to size of human mouth and volume of liquid saliva on its surface relatively small amount of glycoinhibitors is enough to obtain saturating rating concentrations of pathogen inhibiting receptors in mouth. The typical amounts of receptor active monovalent epitopes varies from about 1 µmol to 1000 µmol of the receptor active glycoinhibitors. In a separate embodiment the present invention about therapheutical composition is also directed to pathogen inhibiting nasal sprays. The nasal sprays can be directed against otitis media or lung infections.

### Topical, washing and cosmetic products

It is realized that the common pathogens can spread on human surfaces like human skin, genital epithelia, hair, household surfaces, and other surfaces in human environment. The oligosacchride sequences according to the present invention are also useful for prevention of the pathogens also in these environments. It is therefore also preferred to use the oligosaccharide sequences according to the present invention as single substances, as part of single substances, or as composition comprising at least two glycoinhibitors from different groups according to the present invention in topical or cosmetic products, for example as creams, lotions, or gels. It is also preferred to use the substances or compositions according to the present invention products aimed for washing human skin, hair or genital epithelia, (which can be also called as personal hygiene products), or for household surfaces, dishes or clothes. Traditional antibiotics have been aimed for use of household washing solutions, but are not useful because of resistance problems which are not likely with the substances according to the present invention. In preferred embodiment polyvalent glycoinhibitors are used for washing solutions, in another preferred embodiment monovalent glycoinhibitors are used for washing solutions. Preferably the washing solution is aimed for household use, for use in hospitals and hospital environments or washing of food packages. In a separate embodiment the washing solution is aimed for desinfection of medical instrument or surfaces or textiles or patients in hospital environments.

### Food safety products to be applied to foods or feeds, beverages, drinks and water

Besides the therapheutic uses in humans or in animals the invention is also directed to the use of receptors and compositions according to the invention for the prevention of the infections by using the invention to neutralize pathogens or bacteria inside or on surfaces of food products. Glycoinhibitors according to the present invention can for example be applied on the surfaces of meat products or animal bodies, body parts in meat production to prevent the spreading of pathogenic agents. Use of soluble and other polyvalent conjugates to cover and agglutinate the bacteria are preferred. A specific method to be used on a surface of a solid or semi-solid food product involves contacting the bacteria with the glycoinhibitors described by the invention and optionally washing away the pathogen carbohydrate complexes. This kind of method is not acceptable with traditional antibiotics. The glycoinhibitors according to the invention can be also applied to liquid food products or concentrates or powder to make these including milk and liquid milk like products, various beverages including juices, soft drinks, sport drinks, alcoholic beverages and the like.

In a specific embodiment the glycoinhibitors according to the invention in polymeric form are applied to a liquid food product or a beverage product, potential pathogens are agglutinated by the polyvalent conjugate and the agglutinated complex is removed by a method based on size or solubility of the complex. Non-soluble agglutinates can be removed when these precipitate by standard methods like decanting the solution above the precipitate or more usually more effectively by filtration methods.
Filtration methods can be used to remove larger agglutinated complexes.

Preferred foods to be treated with glycoinhibitors according to the invention includes various animal food products, especially meat products and middle products in processing. Many pathogens including diarrhea causing *E*. *coli* bacteria are transmitted effectively from vegetables, fruits, salads and other plant foods which are not properly washed. The food stuffs needing washing, but not washed properly or washed with contaminated water are especially problematic in developing countries. The present invention is also directed to methods for increasing food safety of plant foods and other foods in need of washing to control the amount of pathogens, especially pathogenic *E. coli* bacteria in the food products. The invention is especially directed to home customer products and products aimed for the food industry to prevent infections from food. The product is preferentially in solid form as powder or pill or in a capsule containing solutions of the receptors according to the invention, which can be applied to food under processing. Such product can be used to prevent diarrheas in developing countries especially diarrheas in children. The food safety product is also directed to the prevention of travellers diarrheas.
The food safety products and feed safety products below can be considered as novel safe preservatives.

### Filter products to purify beverages and water

Contaminated drinks and water are major cause of gastrointestinal diseases, especially diarrheas.
The receptors according to the present invention can be also used to make filters to purify pathogenic agents, especially toxins or toxic lectins or bacteria from liquid food and beverages and water, especially water used for drinking and preparing foods.

Preferentially at least two receptor structures are used. Methods are known to produce solid phase materials to which glycoinhibitors are conjugtated to be used as filters for example from cellulose or plastics or agarose and similar materials. The filters according to the invention also includes affinity chromatography material known in the art. Methods to remove bound materials from such filters are known and in a specific embodiment the filter is regenerated by removing the contaminant and optionally sterilizing the filter by heat or other sterilizing means.

### Feed safety products

The food safety products described above can be also applied to animal solid and liquid feeds and drinking water of animals. Preferred target animals to be protected includes pet animals, especially cats and dogs and cattle or farm animal such as cows and other domestic ruminants, pigs, sheep, horses, poultry including for example hens, ducks and turkeys, and rabbits.

### Water, food and feed safety analytics.

Standard analytic and diagnostic methods in combination with the glycoinhibitors according to the invention can be applied to water, beverages, foods and feeds to measure presence pathogens binding to the receptor carbohydrates. The knowledge of the binding specificities of contaminating pathogens can be applied to design of theraphy when patients are infected or to methods for food safety remove or control pathogens as described above.

### Other carbohydrate based interactions which can be inhibited according to the invention

Beside inhibiting different types of adhesin presentations the invention can be also used to inhibit carbohydrate-carbohydrate interactions and carbohydrate-lectin interactions.

The glycoinhibitors inhibit one or several pathogenic agents by binding one or several pathogens and/or by binding the receptors of one or several pathogenic agents. Preferentially at least two types of pathogen inhibiting glycoinhibitors are used and more preferentially at least three types of glycoinhibitors. In other embodiments at least four, five, six , or seven types of glycoinhibitors are used.

In specific theraphies one or several of the types glycoinhibitors are given separately at different time points. This is especially useful when the administration of all the types glycoinhibitors would have negative effects on the normal flora. The separate administration of the therapheutic compositions can be useful also because of effect of nutritional situation in the gastrointestinal tract could change differently the stability of the on the types glycoinhibitors according to the inventionin the gastrointestinal tract.

### Use of the invention together with probiotic bacteria

When the invention is used to inhibit bacterial binding, especially multiple bacterial bindings, also some beneficial bacterial bindings can be prevented. The normal bacterial flora has many important functions for example in the human gastrointestinal system. The destruction of the normal bacterial flora is however an even larger problem with use of traditional antibiotics.

In a separate embodiment at least one, and preferably at least two or at least three types of glycoinhibitors are administered together with a probiotic microbe and/or a prebiotic substance. The probiotic microbe according to the invention represent a non-harmful bacteria with beneficial functions, for example in digestion of food, providing nutrients and vitamins or covering tissue surfaces from pathogenic bacteria. The probiotic bacteria comprise preferentially one or several or multitude of normal bacterial flora. In a preferred embodiment the probiotic bacterium comprise one or several types, strains, or species of lactic acid bacteria.

The prebiotic substance is a substance supporting the normal flora or probiotic microbe. Preferred prebiotic substances include prebiotic carbohydrates, such as galactose oligosaccharides, xylose oligosaccharide, or fructose oligosaccharides used as prebiotic substances, the prebiotic substances also include polysaccharides and fibers with prebiotic activities such as inulin or midified starches. The present invention is also directed to the use of other polysaccharides which are used in food or for nutritional purposes such as chitosan or beta-glucans for example glucan from oats, which are used to reduce cholesterol and fats. In a preferred embodiment one or several types of glycoinhibitors are chosen so that they are also prebiotic substances like carbohydrates with a non-reducing terminal beta linked galactose residue In a preferred form of theraphy
a) pathogens and potentially part of the normal flora are first removed by one or more preferentially at least two types glycoinhibitors according to the invention
b) probiotic microbe and/or prebiotic substance are applied.
Steps 1 and 2 may also be applied in reversed order, preferably with a large amount of the probiotic microbe and/or prebiotic substance and then step one. According to the invention it is also possible to repeat steps 1 and/or 2 several times while varying the order of the steps. Steps 1 and 2 may be applied at the same time. The substances according to the invention can be administered together with probiotic microbe and/or prebiotic substance or alternatively probiotic microbe and/or prebiotic substance can be included in the compositions according to the invention, and then steps 1 and 2 above can be performed simultaneously.

### Nutritional, food and feed uses

Furthermore, it is possible to use the types of glycoinhibitors as part of a nutritional composition including food- and feedstuff. It is preferred to use the receptor oligosaccharide sequences according to the present invention in single substances or as single substances and more preferably in composition comprising at least two types of glycoinhibitors according to the present invention for nutritional compositions, foods or feed stuffs. It is preferred to use the pathogenic agent receptor types of glycoinhibitors as substances or compositions as a part of so called functional or functionalized food. The said functional food has a positive effect on the person's or animal's health by inhibiting or preventing the binding of pathogenic agent to target cells or tissues. The glycoinhibitor substance or composition can be a part of a defined food or functional food composition. The functional food can contain other acceptable food ingredients accepted by authorities such as Food and Drug Administration in the USA. The glycoinhibitor substance or composition can also be used as a nutritional additive, preferably as a food or a beverage additive to produce a functional food or a functional beverage. The food or food additive can also be produced by having ,e.g., a domestic animal such as a cow or other animal produce glycoinhibitor substance or composition in larger amounts naturally in its milk. This can be accomplished by having the animal overexpress suitable glycosyltransferases in its milk. A specific strain or species of a domestic animal can be chosen and bred for larger production of the glycoinhibitor substance or composition. The glycoinhibitor substance or composition for a nutritional composition or nutritional additive can also be produced by a micro-organisms such as a bacteria or a yeast.

In a separate embodiment glycoinhibitors are also targeted for inhibition of harmful or toxic lectins in food stuffs or feeds. For example lectin concanavalin a reduces the value of jack beans in animal foods. Some lectins such as ricin lectin are very toxic. Preferentially the plant lectin or toxin or bacterial toxin recognizes an oligosaccharide consisting of terminal GlcNAc, Glc, Xyl, Fru, Gal, GalNAc, NeuNAc, Man, or Fuc. More preferentially the plant lectin or tocxin or bacterial toxin recognizes monosaccharide glycoinhibitors according to present invention oligosaccharide sequences for example ManadMan, GlcαdGlc, Glcβ3Glc, Glcβ6Glc, GlcNAcβ6Gal(NAc)ₑ, GlcNAcβ3Gal, GlcNAcβ4GlcNAc, GalNAcβGlcNAc, Galβ4GlcNAc, Galα3Galβ4Glc/Gl*cNAc, GalNAcβGal, GalNAcα3Gal, Galβ3Gal, Galα3Galβ4Gal, Galα4Gal, NeuNAcα3Gal, NeuNAcα6Gal, Fucα2Gal, Fucα3GlcNAc, Fucα6GlcNAc, wherein d is 2, 3, or 6, e is 0 or 1. The glycoinhibitors have the same sequences or mimic the terminal part of lectin or toxin binding structures.

It is especially useful to have the glycoinhibitor substance or composition as part of a food for an infant, preferably as a part of an infant formula. Many infants are fed by special formulas in replacement of natural human milk. The formulas may lack the special lactose based oligosaccharides of human milk. Sialylated and/or fucosylated glycoinhibitors described as pathogen receptors according to the present invention, are also preferred for functional foods and infant formulas. It is preferred to use combinations comprising at least two types of the glycoinhibitor. Diarrhea causing *Escherichia coli* is especially infective with regard to infants or young children, and considering the diseases it may later cause it is reasonable to prevent the infection.

Preferred concentrations for human milk glycoinhibitors in functional food to be consumed (for example, in reconstituted infant formula) are similar to those of total oligosaccharide concentrations present in natural human milk. Alternatively, the total concentration of the glycoinhibitor saccharides used in functional food is the same or similar to the total concentration of natural human milk saccharides, which have the same terminal monosaccharide residues as the glycoinhibitor substances or composition described.

Infant formulas also comprise, beside substances or compositions according to the present invention, other substances used in infant formulas such as fractions from ruminant milks such as proteins from whey or soy protein preparations or protein hydrolysates. The infant formula may also comprise other carbohydrates useful or accepted for infant formulas such as lactose or galactose oligosaccharides.

The glycolipid structures are naturally presented in a polyvalent form on cellular membranes. This type of representation can be mimicked by the solid phase assay described below or by making liposomes of glycolipids or neoglycolipids.

### Preferred conjugates

The present novel neoglycolipids produced by reductive amination of hydrophobic hexadecylaniline were able to provide effective presentation of the oligosaccharides. Most previously known neoglycolipid conjugates used for binding of bacteria have contained a negatively charged groups such as phosphor ester of phosphadityl ethanolamine neoglycolipids. Problems of such compounds are negative charge of the substance and natural biological binding involving the phospholipid structure. Negatively charged molecules are known to be involved in numerous non-specific bindings with proteins and other biological substances. Moreover, many of these structures are labile and can be enzymatically or chemically degraded. The present invention is specifically directed to the non-acidic conjugates of oligosaccharide sequences meaning that the glycoinhibitors are linked to non-acidic chemical structures. Preferably, the non-acidic conjugates are neutral meaning that the glycoinhibitors are linked to neutral, non-charged, chemical structures or alternatively positively charged polymers such as chitosan. The negative charge of the polyvalent conjugate can be eliminated by chemical modification. It is noted that the glycoinhibitor may comprise negatively charged group, e.g. carboxylic acid group, or phosphate or sulphate ester neede fo the function of the conjugate. The preferred conjugates according to the invention are polyvalent substances.

In the previous art bioactive oligosaccharide sequences are often linked to carrier structures by reducing a part of the receptor active oligosaccharide structure. Hydrophobic spacers containing alkyl chains (-CH₂-)ₙ and/or benzyl rings have been used. However, hydrophobic structures are in general known to be involved in non-specific interactions with proteins and other bioactive molecules.

The neoglycolipid data of the examples below show that under the experimental conditions used in the assay the hexadecylaniline parts of the neoglycolipid compounds do not cause non-specific binding for the studied bacterium. In the neoglycolipids the hexadecylaniline part of the conjugate forms probably a lipid layer like structure and is not available for the binding. The invention shows that reducing a monosaccharide residue belonging to the binding epitope may destroy the binding. It was further realized that a reduced monosaccharide can be used as a hydrophilic spacer to link a receptor epitope and a polyvalent presentation structure. According to the invention it is preferred to link the bioactive glycoinhibitor via a hydrophilic spacer to a polyvalent or multivalent carrier molecule to form a polyvalent or oligovalent/multivalent structure. All polyvalent (comprising more than 10 bioactive glycoinhibitors) and oligovalent/multivalent structures (comprising 2-10 bioactive glycoinhibitors) are referred here as polyvalent structures, though depending on the application oligovalent/multivalent constructs can be more preferred than larger polyvalent structures. The hydrophilic spacer group comprises preferably at least one hydroxyl group. More preferably the spacer comprises at least two hydroxyl groups and most preferably the spacer comprises at least three hydroxyl groups.

According to the invention it is preferred to use polyvalent conjugates in which the hydrophilic spacer group linking the glycoinhibitors to polyvalent presentation structure is preferably a flexible chain comprising one or several -CHOH- groups and/or an amide side chain such as an acetamido -NHCOCH₃ or an alkylamido. The hydroxyl groups and/or the acetamido group also protects the spacer from enzymatic hydrolysis in vivo. The term flexible means that the spacer comprises flexible bonds and do not form a ring structure without flexibility. A reduced monosaccharide residues such as ones formed by reductive amination in the present invention are examples of flexible hydrophilic spacers. The flexible hydrophilic spacer is optimal for avoiding non-specific binding of neoglycolipid or polyvalent conjugates. This is essential optimal activity in bioassays and for bioactivity of pharmaceuticals or functional foods, for example.

A general formula for a conjugate with a flexible hydrophilic linker has the following Formula 2:

[GI -O- (X)ₙ-L₁-CH(H/{CH₁₋₂OH}ₚ₁) - {CH₁OH}ₚ₂- {CH(NH-R)} p₃ - {CH₁OH} ₚ₄- L₂]ₘ-Z

wherein L₁ and L₂ are linking groups comprising independently oxygen, nitrogen, sulphur or carbon linkage atom or two linking atoms of the group forming linkages such as -O-, - S-, -CH₂-, -N-, -N(COCH3)-, amide groups -CO-NH- or NH-CO- or N-N- (hydrazine derivative) or amino oxy-linkages -O-N- and N-O-.
GI is glycoinhibitor. X is an optional spacer carbohydrate.
L1 is linkage from carbon 1 of the reducing end monosaccharide of X or when n =0, L1 replaces -O- and links directly from the reducing end C1 of GI.

p1, p2, p3, and p4 are independently integers from 0-7, with the proviso that at least one of p1, p2, p3, and p4 is at least 1. CH₁₋₂OH in the branching term {CH₁₋₂OH}ₚ₁ means that the chain terminating group is CH₂OH and when the p1 is more than 1 there is secondary alcohol groups -CHOH-linking the terminating group to the rest of the spacer. R is preferably acetyl group (-COCH₃) or R is an alternative linkage to Z and then L₂ is one or two atom chain terminating group, in another embodiment R is an analog forming group comprising C₁₋₄ acyl group (preferably hydrophilic such as hydroxy alkyl) comprising amido structure or H or C₁₋₄ alkyl forming an amine. And m > 1 and Z is polyvalent carrier.

In the present invention the glycoinhibitor is preferably linked in a polyvalent or an oligovalent form to a carrier which is not a protein or peptide to avoid antigenicity and possible allergic reactions, preferably the backbone is a natural non-antigenic polysaccharide.

### Diagnostic and analytical uses related to therapheutical uses

Furthermore, it is possible to use the pathogenic agent binding glycoinhibitor according to the present invention in the diagnosis of a condition caused by a pathogenic agent. Diagnostic uses also include the use of the glycoinhibitor for typing of pathogenic agents. The typing of pathogenic agents with regard to binding of the carbohydrate receptors according to the present invention can be used to determine effective combination of therapheutic carbohydrates for a specific pathogenic agents. This can be useful for making specific lower cost theraphies for local infections, the profiles of carbohydrate bindings of major pathogenic agents may differ in different geographic locations and during epidemies.

The term "purified fraction" used herein relates purified or isolated oligosaccharide fraction from natural or synthetic sources. In a preferred embodiment the amount of the active oligosaccharide sequnce or oligosaccharide sequences is analysed and/or controlled from the fraction, optionally the amounts of other related carbohydrate structures are also analysed. The purified fraction has reduced amount of inactive substances originating from the source of the fraction, for example protein, monosaccharide precursors, lactose, or fat. Potentially harmful substances, such as harmful chemicals from synthesis, allergenic proteins, or substances considered ethically harmful, for example by religious or diet culture reasons, are removed to a level where these are not harmful in the final product. For medical use the purified fraction is preferably essentially pure (i.e. a purity of 98 % or better), or non-relevant substances are controlled and comprise preferably at least less than half of the mass of the purified fraction, more preferably less than 20% of the mass of the purified fraction and most preferably less than 5 % of the mass of the purified fraction. In a preferred embodiment of the invention, the production of the purified fraction from animal milk or milks involves at least partial removal of milk protein and/or fat. The purification may comprise filtration methods, such as gelfiltration or ultrafiltration, as well as drying and/or concentrating steps. For non-medical use the purified fraction is preferably essentially pure or the non-relevant substances comprise preferably at least less than 95 % of the mass of the purified fraction, more preferably less than 75% of the mass of the purified fraction and most preferably less than 25 % of the mass of the purified fraction. The purified fraction may be used as such or together with other ingredients of the desired product.

The aim of the invention is to prevent carbohydrate recognizing and/or degrading proteins like ones originating from pathogenic agents, food or feed stuffs, or like destructive antibodies like autoimmune antibodies or allergy antibodies. The invention aims to remove the pathogenic carbohydrate recognizing proteins or inhibit the effect of these.

The present invention is also directed to the use of oligosaccharide sequences which serve as receptors for pathogenic agents. These can be used together with the glycoinhibitors, the glycoinhibitors can prevent the degradation of the glycoinhibitors by glycosidases from pathogenic agent or from food stuffs as described by the present invention. The combinations of the oligosaccharide sequences and glycoinhibitors for inhibition simultanouesly several binding specificities of a pathogenic agent is useful especially when oligosaccharides are chosen for inhibition of lectin specificities requiring at least disaccharide epitope and glycoinhibitors are used for binding specificities which can be inhibited by specific monosaccharides or mimetics or analogs thereof.

Glycolipid and carbohydrate nomenclature is according to recommendations by the IUPAC-IUB Commision on Biochemical Nomenclature (Carbohydrate Res. 1998, 312, 167; Carbohydrate Res. 1997, 297, 1; Eur. J. Biochem. 1998, 257, 29).

It is assumed that Gal, Glc, GlcNAc, and Neu5Ac are of the D-configuration, Fuc of the L-configuration, and all the monosaccharide units in the pyranose form. Glucosamine is referred as GlcN or GlcNH₂ and galactosamine as GalN or GalNH₂. Glycosidic linkages are shown partly in shorter and partly in longer nomenclature, the linkages of the Neu5Ac-residues α3 and α6 mean the same as α2-3 and α2-6, respectively, and with other monosaccharide residues α1-3, β1-3, β1-4, and β1-6 can be shortened as α3, β3, β4, and β6, respectively. Lactosamine referres to N-acetyllactosamine, Galβ4GlcNAc, and sialic acid is N-acetylneuraminic acid (Neu5Ac) or N-glycolylneuraminic acid (Neu5Gc) or any other natural sialic acid. Term glycan means here broadly oligosaccharide or polysaccharide chains present in human or animal glycoconjugates, especially on glycolipids or glycoproteins. In the shorthand nomenclature for fatty acids and bases, the number before the colon refers to the carbon chain lenght and the number after the colon gives the total number of double bonds in the hydrocarbon chain.

The present invention is further illustrated in examples, which in no way are intended to limit the scope of the invention:

### EXAMPLES

### Materials and methods

*Materials -* TLC silica gel 60 (aluminum) plates were from Merck (Darmstadt, Germany). All investigated glycosphingolipids were obtained in our laboratory. β-Galactosidase (*Escherichia coli*) was purchased from Boehringer Mannheim (Germany), Ham's F12 medium from Gibco (U.K.), ³⁵S-methionine from Amersham (U.K.) and FCS (fetal calf serum) was from Sera-Lab (England). β4-Galactosidase (*Streptococcus pneumoniae),* β-N-acetylhexosaminidase (*Streptococcus pneumoniae*) and sialidase (*Arthrobacter ureafaciens*) were from Oxford GlycoSystems (Abington, U.K.). The clinical isolates of *Helicobacter pylori* (strains 002 and 032) obtained from patients with gastritis and duodenal ulcer, respectively, were a generous gift from Dr. D. Danielsson, Örebro Medical Center, Sweden. Type strain 17875 was from Culture Collection, University of Göteborg (CCUG).

*Glycosphingolipids.* The pure glycosphingolipids of experiment shown in Fig. 7 were prepared from total acid or non-acid fractions from the sources listed in Table 2 as described in (Karlsson, 1987). In general, individual glycosphingolipids were obtained by acetylation (Handa, 1963) of the total glycosphingolipid fractions and separated by repeated silicic acid column chromatography, and subsequently characterized structurally by mass spectrometry (Samuelsson *et al.,* 1990), NMR (Falk *et al.,* 1979a,b,c; Koerner Jr *et al.,* 1983) and degradative procedures (Yang and Hakomori, 1971; Stellner *et al.,* 1973). Glycolipids derived from rabbit thymys are described below.

*Purification of glycolipids.* Acid glycosphingolipids were isolated from 1000 g acetone powder of rabbit thymys (Pel-Freez, North Arkansas, Ark. US). The acetone powder was extracted in a Soxhlet apparatus with chroloroform/methanol 2/1 (vol/vol unless otherwise stated) for 24 h followed by chloroform/methanol/water 8/1/1 for 36 h. The extracted lipids, 240 g, were subjected to Folch separation (Folch *et al.,* 1957) and the collected hydrophilic phase to ion-exchange gel chromatography on DE23 cellulose (DEAE, Whatman, Maidstone, UK). These isolation steps gave 2.5 g of acid glycosphingolipids. The gangliosides were separated according to number of sialic acids by ion-exchange gel with open-tubular chromatography on a glass column (50 mm i.d). The column was connected to an HPLC pump producing a concave gradient (pre-programmed gradient no 4, System Gold Chromatographic Software, Beckman Instruments Inc., CA, USA) starting with methanol and ending with 0.5 M CH₃COONH₄ in methanol. The flow rate was 4 ml/min and 200 fractions with 8 ml in each were collected. 300-400 mg of ganglioside mixture was applied at a time to 500 g of DEAE Sepharose, (CL6, Pharmacia, Uppsala, Sweden, bed height approx. 130 mm).

The monosialylated gangliosides were further separated by HPLC on a silica column, 300 mm x 22 mm i.d., 120 Å pore size, 10 µm particle size (SH-044-10, Yamamura Ltd., Kyoto, Japan). Approximately 150 mg of monosialylated gangliosides were applied at time and a streight eluting gradient was used (chloroform/methanol/water from 60/35/8 to 10/103,4 ml/min, 240 fractions).

*Partial acid hydrolysis -* Desialylation of gangliosides was performed in 1.5% CH₃COOH in water at 100°C after which the material was neutralized with NaOH and dried under nitrogen. For partial degradation of the carbohydrate backbone the glycolipid was hydrolyzed in 0.5M HCl for 7 min in a boiling water bath. The material was then neutralized and partitioned in C/M/H₂O, (8:4:3, v/v)^{2.} The lower phase was collected, evaporated under nitrogen and the recovered glycolipids were used for analysis.

*Preparation of pentaglycosylceramide from hexaglycosylceramide by enzyme hydrolysis -* Hexaglycosylceramide (structure 2, Table 1) obtained from heptaglycosylceramide (4 mg, from rabbit thymys) (structure 1, Table 1) by acidic desialylation (see above) was redissolved in C/M (2:1) and applied to a small silica gel column (0.4 x 5 cm). The column was eluted with C/M/H₂O (60:35:8, v/v). Fractions of about 0.2 ml were collected and tested for the presence of carbohydrates. The recovered hexaglycosyleramide (2.0 mg) was dissolved in 1.5 ml of 0.1 M potassium phosphate buffer, pH 7.2, containing sodium taurodeoxycholate (1.5 mg/ml), MgCl₂ (0.001M) and β-galactosidase *(E. coli,* 500 U when assayed with 2-nitrophenyl-β-D-galactoside as a substrate), and the sample was incubated overnight at 37°C. The material was next partitioned in C/M/H₂O (10:5:3) and the glycolipid contained in the lower phase was purified using silica gel chromatography (0.4 x 5 cm columns) as described above for hexaglycosylceramide. To remove all contaminating detergent the chromatography was repeated twice. The final recovery of pentaglycosylceramide was 0.7 mg.

*Endoglycoceramidase digestion of glycolipids (Ito and Yamagata, 1989) -* The reaction mixture contained 200 µg of glycolipid, 80 µg of sodium taurodeoxycholate and 0.8 mU of enzyme in 160 µl of 50 mM acetate buffer, pH 6.0. The sample was incubated overnight at 37°C, after which water (140 µl) and C/M, (2:1, by vol., 1500 µl) were added, and the sample was shaken and centrifuged. The upper phase was dried under nitrogen, redissolved in a small volume of water and desalted on a Sephadex G-25 column (0.4x10 cm), which had been equilibrated in H₂O, and eluted with water. Fractions of about 0.1 ml were collected and tested for the presence of sugars.

*Permethylation of saccharides -* Permethylation was performed according to Larson *et al.,* 1987. Sodium hydroxide was added to samples before methyl iodide as suggested by Needs and Selvendran 1993. In some experiments the saccharides were reduced with NaBH₄ before methylation. In this case the amount of methyl iodide was increased to a final proportion of DMSO (dimethylsulfoxide)/methyl iodide of 1:1 (Hansson and Karlsson, 1990).

*Gas chromatography*/*mass spectrometry -* Gas chromatography was carried out on a Hewlett-Packard 5890A Series II gas chromatograph equipped with an on-column injector and a flame ionization detector. Permethylated oligosaccharides were analyzed on a fused silica capillary column (Fluka, 11m x 0.25 mm i.d.) coated with cross-linked PS264 (film thickness 0.03 µm). The sample was dissolved in ethyl acetate and injected on-column at 80°C. The temperature was programmed from 80°C to 390°C at a rate of 10°C/min with a 2 min. hold at the upper temperature. Gas chromatography-mass spectrometry of the permethylated oligosaccharides was performed on a Hewlett-Packard 5890A Series II gas chromatograph interfaced to a JEOL SX-102 mass spectrometer (Hansson and Karlsson, 1990). FAB-MS analyses were performed on a JEOL SX-102 mass spectrometer. Negative FAB spectra were produced using Xe atom bombardment (10 kV) and triethanolamine as matrix.

*NMR spectroscopy -* Proton NMR spectra were recorded at 11.75 T on a Jeol Alpha 500 (Jeol, Tokyo, Japan) spectrometer. Samples were deuterium exchanged before analysis and spectra were then recorded at 30 °C with a digital resolution of 0.35 Hz/pt. Chemical shifts are given relative to TMS (tetramethylsilane) using the internal solvent signal.

*Analytical enzymatic tests -* Oxford GlycoSystems enzymatic tests were performed according to the manufacturer's recommendations except that Triton X-100 was added to each incubation mixture to final concentration of 0.3%. When a mixture of sialidase and β4-galactosidase were taken for digestion the incubation buffer from β4-galactosidase kit was used. If β-hexosaminidase was present in the digestion mixture the buffer from this enzyme kit was employed. The enzyme concentrations in the incubation mixtures were: 80 mU/ml for Hexβ4HexNAc-galactosidase (*S. pneumoniae*)*,* 120 mU/ml for β-N-Acetylhexosaminidase *(S pneumoniae)* and 1 U/ml for sialidase (*Arthrobacter ureafaciens*) The concentration of substrate was about 20 µM. Enzymatic digestion was performed overnight at 37°C. After digestion the samples were dried and desalted using small columns of Sephadex G-25 (Wells and Dittmer, 1963), 0.3 g, equilibrated in C/M/H₂O (60:30:4.5, by vol.). Each sample was applied on the column in 2 ml of the same solvent and eluted with 2.5 ml of C/M/H₂O, (60:30:4.5) and 2.5 ml of C/M, (2:1). Application and washing solutions were collected and evaporated under nitrogen.

*Other analytical methods -* Hexose was determined according to Dubois *et al.* 1956.

*De-N-acylation.* Conversion of the acetamido moiety of GlcNAc/GalNAc residues into an amine was accomplished by treating various glycosphingolipids with anhydrous hydrazine as described previously (Ångström *et al.,* 1998).

*Bacterial growth.* The *Helicobacter pylori* strains were stored at -80 °C in tryptic soy broth containing 15% glycerol (by volume). The bacteria were initially cultured on on GAB-CAMP agar (Soltesz *et al.,* 1988) under humid (98%) microaerophilic conditions (O₂: 5-7%, CO₂: 8-10% and N₂: 83-87%) at 37 °C for 48-72 h. For labeling colonies were inoculated on GAB-CAMP agar, except for the results presented in Fig.1 where Brucella agar (Difco, Detroit, MI) was used instead, and 50 µCi ³⁵S-methionine (Amersham, U.K.), diluted in 0.5 ml phosphate-buffered saline (PBS), pH 7.3, was sprinkled over the plates. After incubation for 12-24 h at 37 °C under microaerophilic conditions, the cells were scraped off, washed three times with PBS, and resuspended to 1x10⁸ CFU/ml in PBS. Alternatively, colonies were inoculated (1x10⁵ CFU/ml) in Ham's F12 (Gibco BRL, U.K.), supplemented with 10% heat-inactivated fetal calf serum (Sera-Lab). For labeling, 50 µCi ³⁵S_methionine per 10 ml medium was added, and incubated with shaking under microaerophilic conditions for 24 h. Bacterial cells were harvested by centrifugation, and purity of the cultures and a low content of coccoid forms was ensured by phase-contrast microscopy. After two washes with PBS, the cells were resuspended to 1x10⁸ CFU/ml in PBS. Both labeling procedures resulted in suspensions with specific activities of approximately 1 cpm per 100 *Helicobacter pylori* organisms.

*TLC bacterial overlay assay.* Thin-layer chromatography was performed on glass- or aluminum-backed silica gel 60 HPTLC plates (Merck, Darmstadt, Germany) using chloroform/methanol/water 60:35:8 (by volume) as solvent system. Chemical detection was accomplished by anisaldehyde staining (Waldi, 1962). The bacterial overlay assay was performed as described previously (Hansson *et al.,* 1985). Glycosphingolipids (1-4 µg/lane, or as indicated in the figure legend) were chromatographed on aluminum-backed silica gel plates and thereafter treated with 0.3-0.5% polyisobutylmethacrylate in diethylether/*n*-hexane 1:3 (by volume) for 1 min, dried and subsequently soaked in PBS containing 2% bovine serum albumin and 0.1% Tween 20 for 2 h. A suspension of radiolabeled bacteria (diluted in PBS to 1x10⁸ CFU/ml and 1-5x10⁶ cpm/ml) was sprinkled over the chromatograms and incubated for 2 h followed by repeated rinsings with PBS. After drying the chromatograms were exposed to XAR-5 X-ray films (Eastman Kodak Co., Rochester, NY, USA) for 12-72 h.

### RESULTS

The heptaglycosylceramide Neu5Gcα3Galβ4GlcNAcβ3Galβ4GlcNAcβ3Galβ4GlcβCer was purified from rabbit thymus by HPLC as described above. The structure was characterized by NMR and mass spectrometry (data not shown). The heptasaccharide ganglioside was bound by most *Helicobacter pylori* isolates (about 60) tested in the laboratory of the inventors.

In order to detect possible minor isomeric components in the heptaglycosylceramide material, the ganglioside was desialylated, treated with endoglycoceramidase after which the released oligosaccharides were permethylated and analyzed by gas chromatography and EI/MS, (Fig. 1). Two saccharides were identified in the six-sugar region which showed the expected carbohydrate sequence of Hex-HexNAc-Hex-HexNAc-Hex-Hex, as confirmed by fragment ions at *m*/*z* 219, 464, 668, 913 and 1118. When the carbohydrates were converted to alditols (by reduction with NaBH₄) before methylation distinct fragment ions at *mlz* 235, 684 and 1133 were found in addition to the previously listed ions (data not shown). The predominant saccharide, which accounted for more than 90% of the total material (peak B, Fig. 1), was characterized by a strong fragment ion at *m*/*z* 182 confirming the presence of β4GlcNAc (neolacto series, type 2 carbohydrate chain). The minor saccharide (peak A, Fig.1) gave a spectrum typical for type-1 chain (lacto series) with a very weak fragment ion at m/z 182 and a strong fragment ion at *m*/*z* 228. The preparation also contained traces of other sugar-positive substances which might be 4- and 5-sugar-containing saccharides of the same series. Fucose-containing saccharides were not found in the mixture. The purity of the asialoganglioside was tested also by FAB/MS and NMR spectroscopy. The negative FAB/MS of the hexaglycosylceramide (Fig.2, A) confirmed the predicted carbohydrate sequence and showed that the ceramides were composed mainly of sphingosine and C16:0 fatty acid (*m*/*z* 536.5). The NMR spectrum obtained of hexaglycosylceramide (Fig. 3, A) showed four major doublets in the anomeric region with β-couplings (J~8 Hz). They had an intensity ratio of 2:2:1:1. The signals at 4.655 ppm (GlcNAcβ3), 4.256 ppm (internal Galβ4), 4.203 ppm (terminal Galβ4) and 4.166 ppm (Glcβ) were in agreement with results previously published for nLcOse₆-Cer *(Clausen et al.,* 1986). There was also a small doublet at 4.804 ppm, which together with a small methyl signal at 1.81 ppm (seen as a shoulder on the large type 2 methyl resonance) indicated the presence of a small fraction of type 1 chain. Due to the overlap in the 4.15 to 4.25 ppm region the position and distribution of this type 1 linkage could not be determined. The total amount of type 1 linkage was roughly 10%. As the amount of type 1 chain in the pentaglycosylceramide obtained from hexaglycosylceramide by β-galacosidase digestion also was approximately 5% (Fig 3, B) it seems likely that the type 1 linkage was evenly distributed between the internal and external parts of the saccharide chain, i.e. 5% of the glycolipids could be type1-type1.

To find out if the binding activity of the glycolipid was associated with the predominant neolacto (type 2) structure the asialo-glycolipid was treated with β4-galactosidase and β-hexosaminidase, and the products were investigated by TLC and by overlay tests (Fig. 4). As expected, the first enzyme converted the hexaglycosylceramide to a pentaglycosylceramide (A, lane 3) and the mixture of the two enzymes degraded the material to lactosylceramide (B, lane 6). According to visual evaluation of the TLC plates both reactions were complete or almost complete. The same results were obtained for sialidase- and acid-treated material. The β4-galactosidase degradation of hexaglycosylceramide was accompanied by disappearance of the *Helicobacter pylori* binding activity in the region of this glycolipid on TLC plates with simultaneous appearance of a strong activity in the region of pentaglycosylceramides (C, lane 3). Further enzymatic degradation of the pentaglycosylceramide resulted in the disappearance of binding activity in this region. Appearance of binding activity in the four-sugar region was not observed. The sensitivity of the chemical staining of TLC plates is too low to allow trace substances to be observed.

In a separate experiment the parent ganglioside was subjected to partial acid degradation and the released glycolipids were investigated for *Helicobacter pylori* binding activity. Fig. 5 shows TLC of the hydrolyzate (A) and the corresponding autoradiogram (B) after overlay of the hydrolyzate with ³⁵S-labeled *Helicobacter pylori.* Glycolipids located in the regions of hexa-, penta-, tetra- and diglycosylceramides displayed binding activity, whereas triglycosylceramide was inactive.

The binding of the hexa-, penta-, tetraglycosylceramides were similar when tested with at least three *Helicobacter pylori* strains (17875, 002 and 032).

The strongly binding pentaglycosylceramide produced after detachment of the terminal galactose from hexaglycosylceramide and purification by silica gel chromatography was investigated in greater detail. The negative ion FAB/MS spectrum of this glycolipid confirmed a carbohydrate sequence of HexNAc-Hex-HexNAc-Hex-Hex- and showed the same ceramide composition as the hexaglycosylceramide (Fig 2, B). The proton NMR spectrum obtained for the pentaglycosylceramide (Fig. 3, B) had five major β-doublets in the anomeric region: at 4.653 ppm (internal GlcNAcβ3), 4.615 ppm (terminal GlcNAcβ3), 4.261 ppm (double intensity, internal Galβ4), 4.166 (Glcβ), consistent with GlcNAcβ3Galβ4GlcNAcβ3Galβ4GlcβCer and also in perfect agreement with the six sugar compound having been stripped of its terminal Gale. There is also a small β-doublet at 4.787 ppm corresponding to 3-substituted GlcNAcβ3 (type 1 chain). The expected methyl signal was also seen as a shoulder on a much larger methyl signal at 1.82 ppm, but overlap prohibits quantitation of these signals. From the integral of the anomeric proton it can be calculated that 6% of the glycolipid contained type 1 chain. Thus the relative proportion of type 2 and type 1 carbohydrate chains was similar to that of the six sugar glycolipid. The two spots visible on TLC plates both in the hexa- and pentaglycosyl fractions reflected a ceramide heterogeneity rather than differences in sugar chain composition as judged by their susceptibility to β4-galactosidase. The upper penta-region spot appeared both after unselective hydrolysis of the asialoganglioside and selective splitting of 4-linked galactose from the asialoproduct. Furthermore, when hexaglycosylceramide with a high content of the upper chromatographic subfraction was degraded by β4-galactosidase and β-hexosaminidase the resulting lactosylceramide gave two distinct chromatographic bands. Chromatographically homogenous hexaglycosylceramide resulted in only one lactosylceramide band. Both upper and lower sub fractions in the penta-region were highly active as shown by overlay tests.

Glycosphingolipids of the neolacto series with 6, 5 and 4 sugars (structures 2, 4 and 5, Table I) were examined by semi-quantitative tests using the TLC overlay procedure. The glycolipids were applied on silica gel plates in series of dilutions and their binding to *Helicobacter pylori* was evaluated visually after overlay with labeled bacteria and autoradiography ( Fig. 6). The most active species was pentaglycosylceramide, which gave a positive response on TLC plates in amounts down to 0.039 nmol/spot (mean value calculated from 7 experiments, standard deviation δₙ₋₁ = 0.016 nmol). Hexa- and tetraglycosylceramides bound *Helicobacter pylori* in amounts of c:a 0.2 and 0.3 nmoles of glycolipid/spot, respectively.

The binding *of Helicobacter pylori* to higher glycolipids of the investigated series was highly reproducible. The binding frequency for *Helicobacter pylori,* strain 032, recorded for pentaglycosyl- and hexaglycosylceramides was ~ 90% (total number of plates was about 100).

*Binding assays revealing the isoreceptors and specificity of the binding (**Fig 7**.)* In addition to the seven-sugar glycosphingolipid from rabbit thymus having a neolacto core, Neu5Gcα3Galβ4GlcNAcβ3Galβ4GlcNAcβ3Galβ4GlcβCer, and tetra- to hexaglycosylceramides derived thereof, the binding specificity could involve other glycolipids from the neolacto series.

The binding *of Helicobacter pylori* (strain 032) to purified glycosphingolipids separated on thin-layer plates using the overlay assay is shown in Fig. 7. These results together with those from an additional number of purified glycosphingolipids are summarized in Table 2. The binding of *Helicobacter pylori* to neolactotetraosylceramide (lane 1) and the five-and six-sugar glycosphingolipids (lanes 5 and 6) derived from Neu5Gcα3Galβ4GlcNAcβ3Galβ4GlcNAcβ3Galβ4GlcβCer is identical to results above. Unexpectedly, however, binding was also found for GalNAcβ3Galβ4GlcNAcβ3Galβ4GlcβCer (x₂ glycosphingolipid, lane 7) and the defucosylated A6-2 glycosphingolipid GalNAcα3Galβ4GlcNAcβ3Galβ4GlcβCer (no. 12, Table 2). Together with the finding that Galα3Galβ4GlcNAcβ3Galβ4GlcβCer (B5 glycosphingolipid, lane 2) also is binding-active, these results suggest the possibility of cross-binding rather than the presence of multiple adhesins specific for each of these glycosphingolipids (see below). Furthermore, the only extension of the different five-sugar-containing glycosphingolipids just mentioned that was tolerated by the bacterial adhesin was Galβ4 to the thymus-derived GlcNAcβ3-terminated compound (lane 6). Other elongated structures, as the Neu5Ac-x₂ (lane 8) and GalNAcβ3-B5 (no. 25, Table 2), were thus all found to be non-binding. It may be further noticed that the acetamido group of the internal GlcNAcβ3 in B5 is essential for binding since de-*N*-acylation of this moiety by treatment with anhydrous hydrazine leads to complete loss of binding (lane 3) as is the case also when neolactotetraosylceramide is similarly treated (no. 6, Table 2).

As mentioned above, the fact that there are four binding-active five-sugar glycosphingolipids (nos. 10-13, Table 2), all having a neolacto core, suggests that cross-binding to the same adhesin site may be the reason behind these observations.

*Delineation of the neolacto binding epitope.* The relative binding strength of the structures obtained by chemical and enzymatic degradation of the rabbit thymus seven-sugar compound (nos. 1, 5, 10, and 21, Table 2) suggest that the three-sugar sequence GlcNAcβ3Galβ4GlβNAcβ3 may constitute the minimal binding sequence. Thus, in the six-sugar compound an inhibitory effect from the terminal Galβ4 is expected, whereas for neolactotetraosylceramide lack of a terminal GlcNAcβ3 reduces the binding strength since only two out of three sugars in the epitope are present. The essentiality of the internal GlcNAcβ3 is clearly shown by the loss of bacterial binding both to neolactotetraosylceramide and B5 following de-*N*-acylation of the acetamido group to an amine (nos. 6 and 14, Table 2). This non-binding may occur either by loss of a favorable interaction between the adhesin and the acetamido moiety and/or altered conformational preferences of these glycosphingolipids. However, it is difficult to envision a situation where an altered orientation of the internal Galβ4 would sterically hinder access to the binding epitope. Thus, having established that the minimal binding sequence must encompass the GlcNAcβ3Galβ4GlcNAcβ3 sequence it is now easy to rationalize the absence of binding for P₁, H5-2 and the two sialylparagloboside structures (nos. 15, 18-20, Table 2) since these extensions interfere directly with the proposed binding epitope. Also the glycosphingolipid from bovine buttermilk (Teneberg *et al.,* 1994), which has a β6-linked branch of Galβ4GlcNAcβ attached to the internal Galβ4 of neolactotetraosylceramide (no. 26, Table 2), is non-binding due to blocked access to the binding epitope.

Elongation of the different binding-active five-sugar sequences in Table 2 shows that only addition of Galβ4 to the thymus-derived structure is tolerated, in accordance with the observation that the 4-OH position may be either equatorial or axial, but with an ensuing loss of binding affinity due to steric interference. Addition of either Neu5Acα3 to x₂ or GalNAcβ3 to B5 thus results in complete loss of binding (nos. 24 and 25, Table 2). It is further seen that the negative influence of a Fucα2 unit as in H5-2 is confirmed by the non-binding *of Helicobacter pylori* both to A6-2 and B6-2 (nos. 22 and 23, Table 2). Concerning the elongated structure (no. 28, Table 2), terminated by the same trisaccharide found in B5, it must, as in B5, be this terminal trisaccharide that is responsible for the observed binding although a second internal binding epitope also is present. However, binding to the internal epitope can most likely be excluded since the penultimate Galβ4 would be expected to reduce the binding strength similarly to what is observed for the six-sugar compound from rabbit thymus relative to the five-sugar structure. It should also be pointed out that the sialic acid residue of the seven-sugar compound from rabbit thymus does not have an influence on the binding for the bacterial strains used in this study and must consequently be outside the epitope area. Whether sialic acid-dependent or - independent binding *of Helicobacter pylori* is obtained or not depends, however, both on the type of strain and growth conditions (Miller-Podraza *et al.,* 1996,1997a,b).

To summarize, the binding epitope of the neolacto series of glycosphingolipids has to involve the three-sugar sequence GlcNAcβ3Galβ4GlcNAcβ3 in order to obtain maximal activity. From a comparison of the binding pattern of the potential isoreceptors used in this study it can be deduced, that nearly all of this trisaccharide is important for binding to occur, excepting the acetamido group of the terminal GlcNAcβ3 and the 4-OH on the same residue, which are non-crucial. Molecular modeling results confirming the binding specificity are to be published separately.

### Example about use of β3galactosidase to produce a receptor analog.

Hexasaccharide Galβ3GlcNAcβ3Galβ4GlcNAcβ3Galβ4Glc (1 mg, from Dextra labs, UK)) was treated with 400 mU β3/6-galactosidase (Calbiochem., CA, USA) overnight as suggested by the producer. 0.6 mg of pentasaccharide was obtained after HPLC-purification steps, the saccharide was more than 98 % pure when analyzed by mass spectrometry. Part of the pentasaccharide and maltoheptaose (Sigma, Saint Louis, USA) were reductively aminated with 4-hexadecylaniline (abbreviation HDA, from Aldrich, Stockholm, Sweden) by cyanoborohydride (Halina Miller-Podraza, to be published later). The products were characterized by mass spectrometry and were confirmed to be GlcNAcβ3Galβ4GlcNAcβ3Galβ4Glc(red)-HDA and maltoheptaose(red)-HDA [where "(red)-" means the amine linkage structure formed by reductive amination from the reducing end glucoses of the saccharides and amine group of the hexadecylaniline (HDA)]. The compound GlcNAcβ3Galβ4GlcNAcβ3Galβ4Glc(red)-HDA had observable binding activity with regard to *Helicobactei-pylori* in TLC overlay assy described above while the maltoheptaose/red)-HDA was totally inactive. The example shows that the tetrasaccharide GlcNAcβ3Galβ4GlcNAcβ3Gal is a structure binding to *Helicobacter pylori.* The reducing end Glc-residue is probably not needed for the binding because the reduction destroys the pyranose ring structure of the Glc-residue.

Examples of glycosidase reactions leading to glycan receptors of pathogenic agents.
a) Neu5Gcα3Galβ4GlcNAcβ3Galβ4GlcNAcβ3Galβ4GlcβCer,
   Neu5Acα3Galβ4GlcNAcβ3Galβ4GlcβCer and
   Neu5Acα3Galβ3GalNAcβ4Galβ4GlcβCer are reacted with α3-sialidase and glycosphingolipids Galβ4GlcNAcβ3Galβ4GlcNAcβ3Galβ4GlcβCer,
   Galβ4GlcNAcβ3Galβ4GlcβCer (synthesis structure 5, Table 2) and
   Galβ3GalNAcβ4Galβ4GlcβCer, respectively, are formed.
b) Galβ4GlcNAcβ3Galβ4GlcNAcβ3Galβ4GlcβCer is reacted with β-galactosidase (e.g. with β-galactosidase from *E.coli* or *Streptococcus penumoniae* (alone, without β-N-acetylhexosaminidase) as described above and
   GlcNAcβ3Galβ4GlcNAcβ3Galβ4GlcβCer is formed.
c) Galβ4GlcNAcβ3Galβ4GlcNAcβ3Galβ4GlcβCer is treated with β-galactosidase and β-N-acetylhexosaninidase together as described above and Galβ4GlcβCer is formed.
d) Galβ4(Fucα3)GlcNAcβ3Galβ4GlcβCer is treated with α-fucosidase (for example α3/4-fucosidase from almond meal) and Galβ4GlcNAcβ3Galβ4GlcβCer is synhesized.
e) Human erythrocytes containing blood group B antigens Galα3(Fucα2)Galβ4GlcNAcβ- on glycoproteins and glycolipids are treated with α-fucosidase from bovine kidney (Boehringer-Mannheim Biochemicals, Indianapolis, Ind.) as described by Krivan *et al.,* 1986. Receptors for toxin A of *Clostridium difficile,* Galα3Galβ4GlcNAcβ-, are formed.

### Examples of use of glycosidase inhibitors.

Inhibition of β-galactosidase enzyme by D-galactono-1,4-lactone (Sigma, St Louis, USA). β-galactosidase from jack beans (EC 3.2.1.23, Sigma, St Louis, USA) is used to cleave terminal galactose from model oligosaccharide Galβ4GlcNAcβ3Galβ4Glc (Sigma, St Louis, USA). It is noted that the transition state analog galactosidase inhibitor is active against many galactosidases (Huber and Brockbank, 1987, actual active form is probably D-galactono-1,5-lactone) and that the jack bean β-galactosidase cleaves similarity other type 2 lactosamines such as e.g. Galβ4GlcNAcβ3Galβ4GlcNAcβ3Galβ4Glc, specific conditions are needed to cleave Galβ3GlcNAc-sequences or glycospingolipids. In Reaction 1, 25 nmol of Galβ4GlcNAcβ3Galβ4Glc is incubated with 100 mU of β-galactosidase from jack beans in 45 µl of 50 mM sodium citrate pH 4.0, at 37°C, for 8 h. Reaction 2 has the same composition, but contains additionally 55 mM D-galactono-1,4-lactone and it is incubated under the same condition for 8 h. The major products from Reaction 1 are Gal and GlcNAcβ3Galβ4Glc, while unreacted substrate is generally not observable by HPLC and mass spectrometric analysis, while Reaction 2 contains besides these typically more than 50% of uncleaved Galβ4GlcNAcβ3Galβ4Glc.

Inhibition of sialidase from *Vibrio cholerae* by Neu5Ac2en (2,3-anhydro-2-deoxy-N-acetylneuraminic acid, DANA) by the method of (Nöhle *et al.,* 1985). The model compound Neu5Acα3Galβ4Glc (1 mM, purified from bovine milk) is incubated with 10 mU of *Vibrio chloerae* silidase (10 µl, Behringwerke, Marburg, Germany) and various concentrations of Neu5Ac2en (1 µM- 10 mM), in 14 mM phosphate buffer pH 5.4 for 30 min. at 37 °C. The reaction level can be determined by a colorimetric assay for released Neu5Ac and by HPLC-analysis. The enzyme is very effectively inhibited by saturating concentrations of Neu5Ac2en and 50 % inhibition of the enzyme is achieved by 15 µM Neu5Ac2en.

**Table 1**

| Structures of glycosphingolipids discussed in the application. The designation is according to recommendations of IUPAC-IUB Joint Commissions on Biochemical Nomenclature (Lipids 1977 12, 455; Eur. J. Biochem. 1998 257, 293). | |
|---|---|
| Structure | Symbol |
| 1. NeuGcα3Galβ4GlcNAcβ3Galβ4GlcNAcβ3Galβ4GlcβCer | VI³NeuGc-nLcOse₆Cer |
| 2. Galβ4GlcNAcβ3Galβ4GlcNAcβ3Galβ4GlβCer | nLcOse₆Cer |
| 3. Galβ3GlcNAcβ3Galβ3GlcNAcβ3Galβ4GlcβCer | LcOse₆Cer |
| 4. GlcNAcβ3Galβ34GlcNAcβ3Galβ4GlcβCer | nLcOse₅Cer |
| 5. Galβ4GlcNAcβ3Galβ4GlcβCer | nLcOse₄Cer |
| 6. Galβ3GlcNAcβ3Galβ4GlcβCer | LcOse₄Cer |

**Table 2**

| Binding of *Helicobacter pylori* to glycosphingolipids separated on thin-layer chromatograms. | | | | | |
|---|---|---|---|---|---|
| No. | Trivial name | Glycosphingolipid structure^{a} | *H. pylori* binding^{b} | Source^{c} | References |
| 1 | Lactotri | GlcNAcβ3Galβ34Glcβ1Cer | - | RT | (Miller-Prodraza *et al.,* 2001) |
| 2 | GgO3 | GalNAcβ4Galβ4Glcβ1Cer | (+) | GPE | (Yamakawa, 1966) |
| 3 | GgO3 (de-*N*-acylated) | GalNH₂β4Galβ4Glcβ1Cer | - | GPE^{e} | (Ångström *et al.,* 1998) |
| 4 | Le^{y}-6 | Fucα2Galβ4(Fucα3)GlcNAcβ3GalcβGlcβ1Cer | - | DSI | (McKibbin *et al.,* 1982) |
| 5 | Neolactotetra | Galβ4GlcNAcβ3Galβ4Glcβ1Cer | (+) | HE^{f} | |
| 6 | Neolactoteha (de-*N*-acylated) | Galβ4GlcNH₂β3Galβ4Glcβ1Cer | - | HE^{e} | |
| 7 | GgO4 | Galβ3GalNAcβ4Galβ4Glcβ1Cer | + | HB^{g} | |
| 8 | GgO4 (de-*N*-acylated) | Galβ3GalNH₂β4Galβ4Glcβ1Cer | - | HB^{e} | (Ångström *et al.,* 1998) |
| 9 | Le^{x}-5 | Galβ4(Fucα3)GlcNAcβ3Galβ4Glcβ1Cer | - | DSI | (Teneberg *et al.;* 1996) |
| 10 | | GlcNAcβ3Galβ4GlcNAcβ3Galβ4Glcβ1Cer | + | RT^{d} | (Miller-Podraza *et al.;* 2001) |
| 11 | x₂ | GalNAcβ3Galβ4GlcNAcβ3Galβ4Glcβ1Cer | + | HE | (Teneberg *et al.,* 1996; Thorn *et al.,* 1992) |
| 12 | | GalNAcα3Galβ4GlcNAcβ3Galβ4Glcβ1Cer | + | HE^{h} | |
| 13 | B5 | Galα3Galβ4GlcNAcβ3Galβ4Glcβ1Cer | + | RE | (Eto *et al.,* 1968) |
| 14 | B5 (de-*N*-acylated) | Galα3Galβ4GlcNH₂β3Galβ4Glcβ1Cer | - | RE^{e} | |
| 15 | P₁ | Galα4Galβ4GlcNAcβ3Galβ4Glcβ1Cer | - | HE | (Naiki *et al.,* 1975) |
| 16 | H5-1 | Fucα2Galβ3GlcNAcβ3Galβ4Glcβ1Cer | - | HM | (Karlsson and Larson, 1981a) |
| 17 | Le^{b}-6 | Fucα2Galβ3(Fucα4)GlcNAcβ3Galβ4Glcβ1Cer | - | HM | (Karlsson and Larson, 1981b) |
| 18 | H5-2 | Fucα2Galβ4GlcNAcβ3Galβ4Glcβ1Cer | - | HE | (Koscielak *et al.,* 1973) |
| 19 | NeuAcα3-SPG | NeuAcα3Galβ4GlcNAcβ3Galβ4Glcβ1Cer | - | HE | (Ledeen and Yu, 1978) |
| 20 | NeuAcα6-SPG | NeuAcα6Galβ4GlcNAcβ3Galβ4Glcβ1Cer | - | HM | (Nilsson *et al.,* 1981) . |
| 21 | | Galβ4GlcNAcβ3Galβ4GlcNAcβ3Galβ4Glcβ1Cer | (+) | RT^{d} | (Miller-Podraza *et al.,* 2001) |
| 22 | A6-2 | GalNAcα3(Fucα2)Galβ4GlcNAcβ3Galβ4Glcβ1Cer | - | HE | (Laine *et al.,* 1974) |
| 23 | B6-2 | Galα3(Fucα2)Galβ4GlcNAcβ3Galβ4Glcβ1Cer | - | HE | (Koscielak *et al.*, 1973) |
| 24 | NeuAc-x₂ | NeuAcα3GalNAcβ3Galβ4GlcNAcβ3Galβ4Glcβ1Cer | - | HE | (Watanabe and Hakomori, 1979) |
| 25 | | GalNAcβ3Galα3Galβ4GlcNAcβ3Galβ4Glcβ1Cer | - | RCC | (Thurin *et al.,* 1989) |
| 26 | | Galβ4GlcNAcβ6(Galβ4GlcNAcβ3)Galβ4Glcβ1Cer | - | BB | (Teneberg *et al.,* 1994) |
| 27 | | NeuGcα3Galβ4GlcNAcβ3Galβ4GlcNAcβ3Galβ4Glcβ1Cer | (+) | RT | (Lanne *et al.,* 2001) |
| 28 | | Galα3Galβ4GlcNAcβ3Galβ4GlcNAcβ3Galβ4Glcβ1Cer | + | RT | (Lanne *et al.,* 2001) |
| 29 | A7-2 | GalNAcα3(Fucα2)Galβ4(Fucα3)GlcNAcβ3Galβ4Glcβ1Cer | - | DSI | (Falk *et al.,* 1979c) |
| 30 | B7-2 | Galα3(Fucα2)Galβ4(Fucα3)GlcNAcβ3Galβ4Glcβ1Cer | - | HE | |

| | | | | | |
|---|---|---|---|---|---|
| Footnotes to Table 2 a The glycosphingolipid shorthand nomenclature follows recent recommendations (Nomenclature of glycoproteins, 1988). b The following abbreviations are used for the glycosphingolipid sources: RT, rabbit thymus; HE, human erythrocytes; RE, rabbit erythrocytes; HM, human meconium; RCC, rat colon carcinoma; BB, bovine buttermilk; DSI, dog small intestine. c Definition of binding strength is as follows: + denotes a significant darkening of the autoradiogram with 4 µg applied on the TLC. plate, (+) indicates a weak to intermediate darkening while a minus sign signifies no binding. d Prepared from No. 27 by mild acid hydrolysis and No. 10 by subsequent treatment with β-galactosidase. e Glycosphingolipid Nos. 3, 6, 8 and 14 were prepared from Nos. 2, 5, 7 and 13, respectively, by treatment with anhydrous hydrazine. f Prepared from no. 19 by neuraminidase treatment. g Prepared by mild acid hydrolysis of GM1 ganglioside from human brain. h Prepared from No. 22 by incubation in 0.05 M HCl at 80°C for 2 h. | | | | | |

### References

Abramson, J. S. and Hudnor, H. R. (1995) Blood, 85, 1615-19.
Abramson, J. S. and Mills, E. L. (1988) Rev. Infect. Dis.,10, 326-341.
Aggarwal, B. B., Eessalu, T. E. and Hass, P. E. (1985) Nature, 318, 665-667.
Andersson, B., Porras, 0., Halson, L.Å., Lagergård, T., and Svanborg-Edén, C. (1986) J. Inf. Dis. 153, 232-7
Ångström, J., Teneberg, S., Abul Milh, M., Larsson, T., Leonardsson, L, Olsson, B.-M., Ölwegård Halvarsson, M., Danielsson, D., Näslund, I., Ljung, Å., Wadström, T. and Karlsson, K.-A. (1998) Glycobiology, 8, 297-309.
Appelmelk, B.J., Faller, G., Clayes, D., Kirchner, T., and Vandenbroucke-Grauls, C.M.J.E. (1998) Immol. Today 19, 296-299.
Ascencio, F., Fransson, L-Å. and Wadström, T. (1993) J. Med. Microbiol., 38, 240-244.
Avenaud, P., Marais, A., Monteiro, L., Le Bail, B., Biolac Saga, P., Balabaud, C., and Mégraud, F. (2000) Cancer 89, 1431-1439.
Axon, A. T. R. (1993) J. of Antimicrobial Chemotherapy, 32, 61-68.
Babior, B. M. (1978) N. Eng. J. Med., 298, 659-668.
Babu, Y.S., Chand, P., Bantia, S., Kotian, P., Dehghani, A., El-Kattan, Y., Lin, T.-H., Hutchison, T.L., Elliot, A.J., Parker, C.D., Ananth, S.L., Horn, L.L., Laver, G.W., and Montgomery, J.A. (2000) J. Med. Chem. 43, 3482-3486.
Blaser, M. J. (1992) Eur. J. Gastroenterol. Hepatol., 4 (suppl 1), 17-19.
Bock. K., Breimer, M. E., Brignole, A., Hansson, G. C., Karlsson, K.-A., Larson, G., Leffler, H., Samuelsson, B. E., Strömberg, N., Svanborg-Edén, C. and Thurin, J. (1985) J. Biol. Chem., 260, 8545-8551.
Borén, T., Falk, P., Roth, K. A., Larson, G. and Normark, S. (1993)Science, 262, 1892-1895.
Breg, J., Kroon-Batenburg, L. M. J., Strecker, G., Montreuil, J. and Vliegenthart, F. G. (1989) Eur. J. Biochem., 178, 727-739.
Cassidy, L. F., Lyles, D. S. and Abramson, J. S. (1989) J. Immunol., 142, 4401-06.
Castronovo, V., Colin, C., Parent, B., Foidart, J.-M., Lambotte, R., and Mahieu, P. (1989) J. Natl. Cancer Inst. 81, 212-216
Chmiela, M., Wadström, T., Folkesson, H., Planeta Malecka, I., Czkwianianc, E., Rechcinski, T., and Rudnicka, W. (1998) Immunol. Lett. 61, 119-125.
Claeys, D., Faller, G., Appelmelk, B.J., Negrini, R., and Kirchner, T. (1998) Gastroenterology 115, 340-347.
Clausen, H., Levery, S.B., Kannagi, R. and Hakomori, S.-i. (1986) J. Biol. Chem., 261, 1380- 1387.
Colanussi, M. L., White, M. R, Crouch, E. and Hartshorn, K. L. (1999) Blood, 93, 2395-2403.
Correa, T.L., Fox, J., Fontham, E., Ruiz, b., Lin, Y., zaula, D., Taylor, N., Mackinley, D., deLima, E., Portilla, H., Zarama, G. (1990) Cancer 66, 596-574.
Daavidson, V. E. and Sanford, B. A. (1981) Infect. Immun., 32, 118-26.
Daigneault, D. E., Hartshorn, K. L., Liou, L. S., Abruzzi, G. M., White, M. R., Oh, S-K and Tauber, A. I. (1992) Blood, 80, 3227-34.
DeCross, A. J. and Marshall, B. J. (1993) Am. J. Med. Sci., 306, 381-392.
Delorme, C., Brüssow, H., Sidoti, J., Roche, N., Karlsson, K-A, Neeser, J-R and Teneberg, S. (2001) J. Virol., 75, 2276-87.
Dooley, C.P. (1993) Curr. Opin. Gastroenterol., 9, 112-117.
Dubois, M., Gilles, K.A.,Hamilton, J.K., Rebers, P.A. and Smith, F. (1956) Analytical Chemistry 28, 350-356.
Dunn, B.E., Cohen, H. and Blaser, M.J. (1997) Clin. Microbiol. Rev.,10, 720-741.
Eto, T., Ichikawa, Y., Nishimura, K., Ando, S. and Yamakawa, T. (1968) J. Biochem. (Tokyo), 64, 205- 213.
Evans, D. G., Evans Jr, D.J., Molds, J. J., and Graham, D. Y. (1988) Infect. Immun., 56, 2896-06
Falk, K.-E., Karlsson, K.-A. and Samuelsson, B. E. (1979a) Arch. Biochem. Biophys., 192, 164-176.
Falk, K.-E., Karlsson, K.-A. and Samuelsson, B. E. (1979b)Arch. Biochem. Biophys., 192, 177-190.
Falk, K.-E., Karlsson, K.-A. and Samuelsson, B. E. (1979c) Arch. Biochem. Biophys., 192, 191-202.
Farsak, B., Yildirir, A., Akyön, Y., Pinar, A., Öç, M., Böke, E., Kes, S., Tokgözoglu, L. (2000) J. clin. Microbiol. 38, 4408-4411.
Folch, J., Lees, M., And Sloane-Stanley, G.H. (1957) J. Biol. Chem. 226,497-509.
Fukuda, M. N., Dell, A., Oates, J. E.., Wu, P., Klock, J. C. and Fukuda, M. (1985) J. Biol. Chem., 260, 1067-1082.
Gubareva, L.V., Kaiser, L., Hayden, F.G. The Lancet (2000) 355, 827-835.
Handa, S. (1963) Jap. J. Exp. Met, 33, 347-360.
Hansson, G. C., Karlsson, K.-A., Larson, G., Strömberg, N. and Thurin, J. (1985) Anal. Biochem., 146, 158-163.
Hansson, G.C. and Karlsson, H. (1990) Methods Enzymol. 193, 733-738.
Huber, R.E. and Brockbank, R.L. (1987) Biochemistry 26, 1526-1531.
Hu, J., Stults, C.L., Holmes, E.H., and Macher, B.A. (1994) Glycobiology 4, 251-7.
Ito, M. and Yamagata, T. (1989) Methods Enzymol., 179, 488-496.
Jassel, S.V., Ardill, J.E.S., Fillmore, D., Bamford, K.B., O'Connor, F.A., and Buchanan, K.D. Q. J. Med. 92,373-377.
Kannagi, R., Levine, P., Watanabe, K. and Hakomori, S.-i. (1982b) Cancer Res., 42, 5249-5254.
Karlsson, K.-A. (1987) Meth. Enzymol., 138, 212-220.
Karlsson, K.-A. (1989) Annu. Rev. Biochem., 58, 309-350.
Karlsson, K.-A. and Larsson, G. (1981a) J. Biol. Chem. 256, 3512-3524.
Karlsson, K.-A. and Larsson, G. (1981b) FEBS Lett., 128, 71-74.
Kerr, J.R., Al-Khattaf, A., Barson, A.J., and Burnie, J.P. (2000) Arch. Child. Dis., 83,429-434.
Koerner Jr, T. A. W., Prestegard, J. H., Demou, P. C. and Yu, R. K. (1983) Biochemistry, 22, 2676- 2687.
Kopitz, J., Mühl, C., Ehrmann, V., Lehmann, C., and Cantz, M. (1997) Eur. J. Cell Biol. 73, 1-9.
Koscielak, J., Piasek, A., Gorniak, H., Gardas, A. and Gregor, A. (1973) Eur. J. Biochem., 37,214-215.
Krivan, H.C., Clark, G.F., Smith, D. F., and Wilkins, T.D. (1986) Infect. Immun. 53, 573-581.
Kumar, V., Kessler, J., Scott, M.E., Patwardhan, B.H., Tanenbaum, S.W., and Flashner, M. (1981) Carbohydrate Res., 123-130.
Laine, R. A., Stellner, K. and Hakomori, S.-i. (1974) Meth. Membr. Biol., 2, 205-244.
Lanne, B., Miller-Podraza, H., Abul Milh, M., Teneberg, S., Uggla, L., Larsson, T., Leonardsson, I., Jovall, P.-Å., Bergström, J. and Karlsson, K.-A. (2000) manuscript in preparation
Larsen, R. D., Rivera-Marrero, C. A., Ernst, L. K., Cummings, R. D. and Lowe, J. B. (1990) J. Biol. Chem., 265, 7055-7061.
Larson, G., Karlsson, H., Hansson, G.C. and Pimlott, W. (1987) Carbohydr. Res., 161, 281-290
Ledeen, R. and Yu, R. K. (1978) Res. Methods Neurochem., 4, 371-410.
Lin, J.-T., Wang, J.-T., Wang, M.-S., Wu, M.-S. and Chen, C.-J. (1993) Hepato-Gastroenterol., 40, 596-599.
Lingwood, C. A., Huesca, M. and Kuksis, A. (1992) Infect. Immun., 60, 2470-2474.
Mayo, S. L., Olafsen, B. D. and Goddard III, W. A. (1990) J. Chem. Phys., 94, 8897-8909.
McGee, C.M., and Murray, D.R. (1986) Ann. Botany 57, 179- .
Meyer, B. (1990) Topics Curr. Chem., 154, 141-208.
Miller-Podraza, H., Abul Milh, M., Bergström, J. and Karlsson, K.-A. (1996) Glycoconj. J., 13, 453-460.
Miller-Podraza, H., Bergström, J., Abul Milh, M. and Karlsson, K.-A. (1997a)Glycoconj. J., 14, 467-471.
Miller-Podraza, H., Abul Milh, M., Teneberg, S. and Karlsson, K.-A. (1997b) Infect. Immun., 65, 2480- 2482.
Miller-Podraza, H., Abul Milh, M., Ångström, J., Jovall. P.-Å., Wilhelmsson, U., Lanne, B., Karlsson, H., and Karlsson, K.-A. (2000) manuscript in preparation.
Mysore, J.V., Wiggington, T., Simon, P.M., Zopf, D., Heman-Ackah, L.M. and Dubois, A. (1999) Gastroenterology, 117, 1316-1325
Naiki, M., Fong, J., Ledeen, R. and Marcus, D. M. (1975) Biochemistry, 14, 4831-4836.
Needs, P.W. and Selvendran, R.R. (1993) Carbohydr. Res., 245, 1-10.
Nilsson, H.-O., Taneera, J., Castedal, M., Glatz, E., Olsson, R., and Wadström, T. (2000) J. Clin. Microbiol. 38, 1072-1076.
Nilsson, O., Månsson, J.-E., Tibblin, E. and Svennerholm, L. (1981)FEBS Lett., 133, 197-200.
Nomenclature of glycoproteins (1988) J. Biol. Chem., 262, 13-18.
Nomura, A. and Stemmermann, G. N. (1993) J. Gastroenterol. Hepatol., 8, 294-303.
Nyholm, P. G., Samuelsson, B. E., Breimer, M. and Pascher, 1. (1989) J. Mol. Recog., 2, 103-113.
Nyholm, P.-G. and Pascher, I. (1993) Biochemistry, 32, 1225-1234.
Nöhle, U., Shukla, A.K., Schröder, C., Reuter, G., Schauer, R., Kamerling, J.P., Vliegenthart, J.F.G. (1985) Eur. J. Biochem. 152, 459-463.
Ofek, I. and Sharon, N. (1988) Infect. Immun., 56, 539-547.
Pakodi, F., Abdel-Salam, O.M.E., Debraceni, A., and Mozsik, G. (2000) J. Physiol. (Paris), 94, 139-152.
Platt, F.M., Neises, G.R., Karlsson, G.B., Dwek, R.A., and Butters, T.D. (1994) J. Biol. Chem. 269, 27108-27114.
Parsonnet, J., Friedman, G.D., Vandersteen, D.P., Chang, Y., Vogelman, J.H., Orentreich, N. and Sibley, R.K. (1991) N. Engl. J. Med., 325, 1127-31.
Plotkowski, M-C, Puchelle, E., Beck, G., Jaquot, J. and Hannoun C. (1986) Am. Rev. Resp. Disease, 134, 1040-44.
Rappé, A. K. and Goddard III, W. A. (1991) J. Chem. Phys., 95,3358-3363.
Rautelin, H., Blomberg, B., Järnerot, G. and Danielsson, D. (1994a) Scand. J. Gastroenterol., 29, 128-132.
Rautelin, H., von Bonsdorff, C.-H., Blomberg, B. and Danielsson, D. (1994b) J. Clin. Pathol., 47, 667-669.
Rebora, R., Drago, F., and Parodi, A. (1995) Dermatology 191, 6-8.
Saitoh, T., Natomi, H., Zhao, W., Okuzumi, K., Sugano, K., Iwamori, M. and Nagai, Y. (1991) FEBS Lett., 282, 385-387.
Samuelsson, B. E., Pimlott, W. and Karlsson, K.-A. (1990) Meth. Enzymol., 193, 623-646.
Sasaki, K., Kurata-Miura, K., Ujita, M., Angata, K., Nakagawa, S., Sekine, S., Nishi, T., and Fukuda, M. (1997) Proc. Natl. Acad. Sci. (USA) 94, 14294-14299.
Schreiner, E., and Zbiral, E. (1985) Carbohydrate Res., 216, 61-66.
Siebert, H.-C., Reuter, G., Schauer, R., von der Lieth, C.-W. and Dabrowski, J. (1992) Biochemistry, 31, 6962-6971.
Simon, P. M., Goode, P. L., Mobasseri, A., and Zopf, D. (1997) Infect. Immun. 65, 750-757
Soltesz, V., Schalen, C. and Mårdh, P. A. (1988) Proceedings of the Fourth International Workshop on Campylobacter Infections (Kaijser, B. and Falsen, E., eds.) pp. 433-436, Goterna, Kungälv, Sweden.
Steininger, H., Faller, G., Dewald, E., Brabletz, T., Jung, A., and Kirchner, T. (1998) Virchows Arch. 433, 13-18.
Stellner, K., Saito, H. and Hakomori, S.-i. (1973) Arch. Biochem. Biophys., 155, 464-472.
Stellner, K. and Hakomori, S.-i. (1974) J. Biol. Chem., 249, 1022-1025.
Stroud, M. R., Handa, K., Salyan, M. E. K., Ito, K., Levery, S. B., Hakomori, S.-i., Reinhold, B. B. and Reinhold, V. N. (1996) Biochemistry, 35, 758-769.
Sung, J., Russell, R.I., Neyomans, Chan, F.K., Chen, S., Fock, K., Goh, K.L., Kullavanijaya, P., Kimura, K., Lau, C., Louw, J., Sollano, J., Triadiafalopulos, G., Xiao, S., Brooks, P. (2000) J. Gastroenterol. Hepatol., 15, Suppl: G58-68.
Teneberg, S., Ångström, J., Jovall, P.-Å. and Karlsson, K.-A. (1994) J. Biol. Chem., 269, 8554-8563.
Teneberg, S., Lönnroth, I., Torres Lopez, J. F., Galili, U., Ölwegård Halvarsson, M., Ångström, J. and Karlsson, K.-A. (1996) Glycobiology, 6, 599-609.
Teneberg, S., Abul Milh, M., Lanne, B., Jovall, P.-Å., Karlsson, H., Ångström, J., Ölwegård Halvarsson, M., Danielsson, D., Ljung, Å., Wadström, T. and Karlsson, K.-A. (2000, manuscript in preparation).
Thorn, J. J., Levery, S. B., Salyan, M. E. K., Stroud, M. R., Cedergren, B., Nilsson, B., Hakomori, S.-i. and Clausen, H. (1992) Biochemistry, 31, 6509-6517.
Thurin, J., Brodin, T., Bechtel, B., Jovall, P.-Å., Karlsson, H., Strömberg, N., Teneberg, S., Sjögren, H. O. and Karlsson, K.-A. (1989) Biochim. Biophys. Acta, 1002, 267-272*.*
Waldi, D. (1962) in Dünnschicht-Chromatographie (Stahl, E., ed.) pp. 496-515, Springer-Verlag, Berlin.
Watanabe, K. and Hakomori, S.-i. (1979) Biochemistry, 18, 5502-5504.
Wells, M.E. and Dittmer, J.C. (1963) Biochemistry, 2, 1259-1263.
Winchester, B., et al. (1990) Biochem. J. 265, 277-.
Wotherspoon, A.C., Doglioni, C., Diss, T.C., Pan, L., Moschini, A., de Boni, M. and Isaacson, P.G. (1993) Lancet, 342, 575-577
Woynarowska, B., Wikiel, H., Sharma, M., Carpenter, N., Fleet, G.W., and Bednacki, R.J. (1992) Anticancer Res. 12, 161-6.
Yamakawa, (1966)
Yang, H.-j. and Hakomori, S.-i. (1971) J. Biol. Chem., 246, 1192-1200.

## Claims

1. Use of at least one glycoinhibitor monosaccharide substance comprising a pyranose formed ring structure selected from the group consisting of GlcNAcβ, GalNAcβ, Galβ, Gala, GalNAcα, Fucα, Mana, and Neu5Acα, wherein said monosaccharide substance is linked to a aglycon, for the manufacture of a medicament or a pharmaceutical, cosmetic, or nutritional composition for the treatment or prophylaxis of an infectious disease.

2. The use according to claim 1, wherein said aglycon is a monosaccharide mimicking structure.

3. The use according to claim 2, wherein said monosaccharide mimicking structure is a polyhydroxyl substance.

4. The use according to claim 1, wherein said aglycon is a hydrophilic linker.

5. The use according to claim 1, wherein said aglycon is a non-monosaccharide spacer linking said glycoinhibitor to a carrier.

6. The use according to claim 1, wherein said glycoinhibitor monosaccharide substance comprising a pyranose formed ring structure is selected from the group consisting of GlcNAcβ, GalNAcβ, Galα, GalNAcα, Fucα, Manα, and Neu5Acα.

7. The use according to any one of claims 1-6, wherein at least one oligosaccharide sequence according to Formula
[Galβy]ₚ[Hex(NAc)ᵣα/βz]ₛGalβ4Glc(NAc)ᵤ (I)
wherein p, r, s, and u are each independently 0 or 1, and y is either linkage position 3 or 4, and z is either linkage position 3 or 4, and Hex is either Gal or Glc,
so that when p is 1 and y=3, then Hex is Galβ or Glcβ and r=1, or p is 1 and y=4 then Hex is Glcβ and r=1, when p is 0 and z is 4, then Hex is Galβ and r is 1 or Hex is Gala and r is 0, is further used with said glycoinhibitor monosaccharide substance for the manufacture of said medicament or a pharmaceutical, cosmetic, or nutritional composition.

8. The use according to any one of previous claims, wherein the glycoinhibitor monosaccharide substance is glycosidically N-, S-, or C-linked.

9. The use according to any one of the claims 1-8, wherein the glycoinhibitor is in monovalent, oligovalent, or polyvalent form.

10. The use according to claim 1, wherein the glycoinhibitor is a-anomeric glycoinhibitor.

11. The use according to claim 10, wherein the glycoinhibitor is NeuNAcα or Fucα

12. The use according to claim 1, wherein the glycoinhibitor substance comprises one or two pyranose formed ring structure selected from the group consisting of GlcNAcβ, GalNAcβ, and Galα.

13. The use according to claim 1, wherein the glycoinhibitor substance comprises a pyranose formed ring structure selected from the group consisting of GlcNAcβ, Manα, and Fucα.

14. The use according to any one of the preceding claims, wherein at least two or three different pyranose formed ring structure glycoinhibitors are used.

15. The use according to claim 1, wherein said disease is caused by the presence of *Helicobacter pylori* or diarrhea causing *E.coli* or *Clostridium difficile* in the gastrointestinal tract of a patient.

16. The use according to claim 15, wherein said disease is caused by *Helicobacter pylori* and is an urinary tract infection, chronic superficial gastritis, gastric ulcer, duodenal ulcer, non-Hodgkin lymphoma in human stomach, gastric adenocarcinoma, heart disease, skin disease, liver disease, pancreatic disease, or sudden infant death syndrome.

17. The use according to claim 1, wherein the glycoinhibitor is for the treatment of influenza or a coinfection of bacterium and influenza virus.

18. The use according to claim 17, wherein the coinfection causes pneumonia or bronchitis.

19. The use according to claim 1 or 17, wherein said disease is a secondary bacterial infectious disease associated with influenza and said glycoinhibitor substance is used together with an oligosaccharide substance defined in Formula I of claim 7.

20. The use according to claim 1, wherein said disease is caused by the infecting bacterium selected from the group consisting of *Streptococcus pneumoniae, Escherichia coli, Klebsiella pneumoniae, Pseudomonas auruginosa, Pseudomonas cepacia, Xanthomonas maltophilia, Haemophilus influenzae, Haemophilus parainfluenzae,* and *Staphylococcus aureus.*

21. The use according to claim 1, wherein said nutritional composition is an infant formula, food preservative or additive.

22. The use according to claim 1, wherein said cosmetic composition is a mouth hygiene product, topical, or washing product.

23. The use according to claim 22, wherein the mouth hygiene product is selected from the group of tooth pastes, mouth wash solutions, tablets, and chewing gums.

24. Use of a composition comprising glycoinhibitors as defined in claims 1-14 for coating surfaces of food products for improved food safety.

25. Use of a composition comprising glycoinhibitors as defined in claims 1-14 for inhibition or agglutination of pathogen *ex vivo.*

26. The use according to claim 25, wherein the pathogen is *E*. *coli.*

27. Use of a composition comprising glycoinhibitors as defined in claims 1-14 as a part of filter material to purify pathogens from liquid food, beverages and water by filtering.

## Patentansprüche

1. Verwendung mindestens einer Glycoinhibitor-Monosaccharidsubstanz umfassend eine aus Pyranose gebildete Ringstruktur ausgewählt aus der Gruppe bestehend aus GlcNAcβ, GalNAcβ, Galβ, Gala, GalNAcα, Fucα, Manα, und Neu5Acα, wobei die Monosaccharidsubstanz mit einem Aglykon gekoppelt ist, für die Herstellung eines Arzneimittels oder einer pharmazeutischen, kosmetischen oder diätischen Zusammensetzung zur Behandlung oder Vorbeugung einer infektiösen Krankheit.

2. Verwendung nach Anspruch 1, wobei das Aglykon eine ein Monosaccharid nachahmende Struktur ist.

3. Verwendung nach Anspruch 2, wobei die das Monosaccharid nachahmende Struktur eine Polyhydroxylsubstanz ist.

4. Verwendung nach Anspruch 1, wobei das Aglykon ein hydrophiler Linker ist.

5. Verwendung nach Anspruch 1, wobei das Aglykon ein Nicht-Monosaccharid-Abstandshalter ist, der den Glycoinhibitor an einen Träger koppelt.

6. Verwendung nach Anspruch 1, wobei die eine aus Pyranose gebildete Ringstruktur umfassende Glycoinhibitor-Monosaccharidsubstanz ausgewählt ist aus der Gruppe bestehend aus GlcNAcβ, GalNAcβ, Gala, GalNAcα, Fucα, Manα, und Neu5Acα.

7. Verwendung nach einem der Ansprüche 1 bis 6, wobei mindestens eine Oligosaccharidsequenz der Formel
[Galβy]ₚ[Hex(NAc)ᵣα/βz]ₛGalβ4Glc(NAc)ᵤ (I),
wobei p, r, s und u jeweils unabhängig voneinander 0 oder 1 sind, und y Verbindungsposition 3 oder 4 ist, und z Verbindungsposition 3 oder 4 ist, und Hex Gal oder Glc ist,
so dass, wenn p 1 und y 3 ist, dann Hex Galβ oder Glcβ und r 1 ist, oder wenn p 1 und y 4 ist, dann Hex Glcβ und r 1 ist, wenn p 0 und z 4 ist, dann Hex Galβ und r 1 ist oder Hex Galα und r 0 ist,
weiter mit der Glycoinhibitor-Monosaccharidsubstanz für die Herstellung des Arzneimittels oder der pharmazeutischen, kosmetischen oder diätischen Zusammensetzung verwendet wird.

8. Verwendung nach einem der vorhergehenden Ansprüche, wobei die Glycoinhibitor-Monosaccharidsubstanz N-, S-, oder C-glycosidisch verbunden ist.

9. Verwendung nach einem der Ansprüche 1 bis 8, wobei der Glycoinhibitor in monovalenter, oligovalenter oder polyvalenter Form vorliegt.

10. Verwendung nach Anspruch 1, wobei der Glycoinhibitor ein α-anomerer Glycoinhibitor ist.

11. Verwendung nach Anspruch 10, wobei der Glycoinhibitor NeuNAcα oder Fucα ist.

12. Verwendung nach Anspruch 1, wobei die Glycoinhibitorsubstanz eine oder zwei aus Pyranose gebildete Ringstrukturen ausgewählt aus der Gruppe bestehend aus GlcNAcβ, GalNAcβ und Gala umfasst.

13. Verwendung nach Anspruch 1, wobei die Glycoinhibitorsubstanz eine aus Pyranose gebildete Ringstruktur ausgewählt aus der Gruppe bestehend aus GlcNAcβ, Manα und Fucα umfasst.

14. Verwendung nach einem der vorhergehenden Ansprüche, wobei mindestens zwei oder drei unterschiedliche aus Pyranose gebildetete Ringstrukturen als Glycoinhibitoren verwendet werden.

15. Verwendung nach Anspruch 1, wobei die Krankheit durch die Anwesenheit von *Helicobacter pylori* oder Diarrhö auslösendem *E.coli* oder *Clostridium difficile* im gastrointestinalen Trakt eines Patienten verursacht wird.

16. Verwendung nach Anspruch 15, wobei die Krankheit durch *Helicobacter pylori* verursacht wird, und eine Infektion des Harntraktes, chronische oberflächliche Gastritis, ein Magengeschwür, ein Dünndarmgeschwür, ein Nicht-Hodgkin Lymphom im menschlichen Magen, ein Magenadenocarcinom, eine Herzkrankheit, eine Hautkrankheit, eine Leberkrankheit, eine Bauchspeicheldrüsenkrankheit oder das Syndrom plötzlicher Kindstod ist.

17. Verwendung nach Anspruch 1, wobei der Glycoinhibitor zur Behandlung von Influenza oder einer Coinfektion von Bakterien und Influenzavirus ist.

18. Verwendung nach Anspruch 17, wobei die Coinfektion Pneumonie oder Bronchitis auslöst.

19. Verwendung nach Anspruch 1 oder 17, wobei die Krankheit eine sekundäre bakterielle, infektiöse, mit Influenza assoziierte Krankheit ist und die Glycoinhibitorsubstanz zusammen mit einer Oligosaccharidsubstanz wie in Formel I aus Anspruch 7 definiert verwendet wird.

20. Verwendung nach Anspruch 1, wobei die Krankheit durch das infizierende Bakterium ausgewählt aus der Gruppe bestehend aus *Streptococcus pneumoniae, Escherichia coli, Klebsiella pneumoniae, Pseudomonas auruginosa, Pseudomonas cepacia, Xanthomonas maltophilia, Haemophilus influenzae, Haemophilus parainfluenzae* und *Staphylococcus aureus* ausgelöst wird.

21. Verwendung nach Anspruch 1, wobei die diätische Zusammensetzung eine Säuglingsnahrung, ein Konservierungsstoff oder ein Zusatzstoff für Nahrungsmittel ist.

22. Verwendung nach Anspruch 1, wobei die kosmetische Zusammensetzung ein Mundhygieneprodukt, ein topisches Produkt oder Reinigungsprodukt ist.

23. Verwendung nach Anspruch 22, wobei das Mundhygieneprodukt ausgewählt ist aus der Gruppe von Zahnpasten, Mundspülungen, Tabletten und Kaugummis.

24. Verwendung einer Zusammensetzung umfassend Glycoinhibitoren wie in den Ansprüchen 1 bis 14 definiert zur Beschichtung von Oberflächen von Nahrungsmittelprodukten zur verbesserten Nahrungsmittelsicherheit.

25. Verwendung einer Zusammensetzung umfassend Glycoinhibitoren wie in den Ansprüchen 1 bis 14 definiert zur Hemmung oder Agglutination eines Pathogens *ex vivo.*

26. Verwendung nach Anspruch 25, wobei das Pathogen *E. coli ist.*

27. Verwendung einer Zusammensetzung umfassend Glycoinhibitoren wie in den Ansprüchen 1 bis 14 definiert als Teil eines Filtermaterials zur Aufreinigung von Pathogenen aus flüssigen Nahrungsmitteln, Getränken und Wasser durch Filtrieren.

## Revendications

1. Utilisation d'au moins une substance monosaccharidique glyco-inhibitrice comprenant une structure cyclique formée de pyranose choisie dans le groupe constitué par GlcNAcβ, GalNAcβ, Galβ, Galα, GalNAcα, Fucα, Manα et Neu5Acα, où ladite substance monosaccharidique est liée à un aglycone, pour la fabrication d'un médicament ou d'un produit pharmaceutique, d'une composition cosmétique ou nutritionnelle pour le traitement ou la prophylaxie d'une maladie infectieuse.

2. Utilisation selon la revendication 1, où ledit aglycone est une structure mimant un monosaccharide.

3. Utilisation selon la revendication 2, où ladite structure mimant un monosaccharide est une substance polyhydroxylée.

4. Utilisation selon la revendication 1, où ledit aglycone est un lieur hydrophile.

5. Utilisation selon la revendication 1, où ledit aglycone est un espaceur non monosaccharidique liant ledit glyco-inhibiteur à un support.

6. Utilisation selon la revendication 1, où ladite substance monosaccharidique glyco-inhibitrice comprenant une structure cyclique formée de pyranose est choisie dans le groupe constitué par GlcNAcβ, GalNAcβ, Galα, GalNAcα, Fucα, Manα et Neu5Acα.

7. Utilisation selon l'une quelconque des revendications 1 à 6, où au moins une séquence oligosaccharidique selon la formule
**[Galβy]ₚ[Hex(NAc)ᵣα/βz]ₛGalβ4Glc(NAc)_{U}** (I)
dans laquelle p, r, s et u valent chacun indépendamment 0 ou 1, et y représente une position de liaison soit en 3 soit en 4, et z représente une position de liaison soit en 3 soit en 4, et Hex représente Gal ou Glc,
de sorte que lorsque p vaut 1 et y = 3, alors Hex représente Galβ ou Glcβ et r = 1, ou p vaut 1 et y = 4 alors Hex représente Glcβ et r = 1, lorsque p vaut 0 et z représente 4, alors Hex représente Galβ et r vaut 1 ou Hex représente Gala et r vaut 0, est en outre utilisée avec ladite substance monosaccharidique glyco-inhibitrice pour la fabrication dudit médicament ou produit pharmaceutique, de ladite composition cosmétique ou nutritionnelle.

8. Utilisation selon l'une quelconque des revendications précédentes, où la substance monosaccharidique glyco-inhibitrice est liée par une liaison N-, S- ou C-glycosidique.

9. Utilisation selon l'une quelconque des revendications 1 à 8, où le glyco-inhibiteur est sous une forme monovalente, oligovalente ou polyvalente.

10. Utilisation selon la revendication 1, où le glyco-inhibiteur est un glyco-inhibiteur anomère α.

11. Utilisation selon la revendication 10, où le glyco-inhibiteur est NeuNAcα ou Fucα.

12. Utilisation selon la revendication 1, où la substance glyco-inhibitrice comprend une structure cyclique formée d'un ou deux pyranoses choisie dans le groupe constitué par GlcNAcβ, GalNAcβ et Galα.

13. Utilisation selon la revendication 1, où la substance glyco-inhibitrice comprend une structure cyclique formée d'un pyranose choisie dans le groupe constitué par GlcNAcβ, Manα et Fucα.

14. Utilisation selon l'une quelconque des revendications précédentes, où au moins deux ou trois glyco-inhibiteurs différents à structure cyclique formée de pyranose sont utilisés.

15. Utilisation selon la revendication 1, où ladite maladie est provoquée par la présence de *Helicobacter pylori* ou de *E. coli* responsable de diarrhée ou de *Clostridium difficile* dans le tube digestif d'un patient.

16. Utilisation selon la revendication 15, où ladite maladie est provoquée par *Helicobacter pylori* et est une infection du système urinaire, une gastrite superficielle chronique, un ulcère gastrique, un ulcère duodénal, un lymphome non hodgkinien dans l'estomac humain, un adénocarcinome gastrique, une maladie cardiaque, une maladie cutanée, une maladie hépatique, une maladie pancréatique ou le syndrome de la mort subite du nourrisson.

17. Utilisation selon la revendication 1, où le glyco-inhibiteur est destiné au traitement de la grippe ou d'une co-infection d'une bactérie et du virus de la grippe.

18. Utilisation selon la revendication 17, où la co-infection provoque une pneumonie ou une bronchite.

19. Utilisation selon la revendication 1 ou 17, où ladite maladie est une maladie bactérienne infectieuse secondaire associée à la grippe et ladite substance glyco-inhibitrice est utilisée conjointement avec une substance oligosaccharidique définie dans la formule I de la revendication 7.

20. Utilisation selon la revendication 1, où ladite maladie est provoquée par la bactérie infectieuse choisie dans le groupe constitué par *Streptococcus pneumoniae, Escherichia coli, Klebsiella pneumoniae, Pseudomonas auruginosa, Pseudomonas cepacia, Xanthomonas maltophilia, Haemophilus influenzae, Haemophilus parainfluenzae* et *Staphylococcus aureus.*

21. Utilisation selon la revendication 1, où ladite composition nutritionnelle est un lait pour nourrisson, un conservateur ou un additif alimentaire.

22. Utilisation selon la revendication 1, où ladite composition cosmétique est un produit d'hygiène buccale, un produit topique ou de lavage.

23. Utilisation selon la revendication 22, où le produit d'hygiène buccale est choisi dans le groupe de pâtes dentifrices, de solutions de bains de bouche, de comprimés et de chewing-gums.

24. Utilisation d'une composition comprenant des glyco-inhibiteurs tels que définis dans les revendications 1 à 14 pour l'enrobage de surfaces de produits alimentaires pour une meilleure sécurité alimentaire.

25. Utilisation d'une composition comprenant des glyco-inhibiteurs tels que définis dans les revendications 1 à 14 pour l'inhibition ou l'agglutination d'un agent pathogène *ex vivo.*

26. Utilisation selon la revendication 25, où l'agent pathogène est *E. coli.*

27. Utilisation d'une composition comprenant des glyco-inhibiteurs tels que définis dans les revendications 1 à 14 comme partie de matériau filtre pour purifier des aliments liquides, des boissons et l'eau d'agents pathogènes par filtration.
